# EUROPEAN PATENT APPLICATION

(11) **EP 4 249 585 A1**
(43) Date of publication of application: **27.09.2023**
(21) Application number: 22164561.7
(22) Date of filing: 25.03.2022
(51) Int. Cl.: C12N 5/00, C12N 5/077

(54) **HEART TISSUE MODELS, METHOD OF PRODUCING HEART TISSUE MODELS AND USES OF HEART TISSUE MODELS**

(71) Applicant: Technische Universität München - TUM, 80333 München (DE)
(72) Inventor: Meier, Anna, Albigny-sur-Saone (FR); Moretti, Alessandra, München (DE); Laugwitz, Karl-Ludwig, München (DE)
(74) Representative: Grund, Martin

(57) **Abstract**

Pluripotent stem cell-derived cardiac organoids have emerged as powerful *in vitro* models of human development and disease, but none have yet demonstrated the formation of a *bona fide* epicardial compartment. The present invention provides cardiac organoids showing self-organization of highly functional ventricular myocardium and epicardium, which were thus called epicardioids. Time course single-cell genomics in the herein described epicardioids revealed principles of human epicardial biology, including lineage heterogeneity and functional crosstalk with other cardiac cell types. In addition, the herein disclosed epicardioids represent an advanced system to model multicellular mechanisms of heart disease. The invention thus also relastes to the use of epicardioids as heart tissue model, a method of generating the epicardioids, and heart tissue models prepared by the disclosed methods.

## Description

### FIELD OF THE INVENTION

The present invention lies in the field of heart tissue models. More specifically, the invention provides for *in vitro* heart tissue models named epicardioids, which match the multilayered structure of the ventricular epicardium and myocardium of human embryos. The epicardioids can be used as a heart tissue model. In a further aspect, the invention provides for a method of producing such a heart tissue model *in vitro.* Moreover, the invention relates to a heart tissue model produced by methods disclosed herein.

### BACKGROUND OF THE INVENTION

Cardiovascular disease (CVD) is the leading cause of death and a major contributor to disability worldwide. Despite this significant public health burden, the development of new cardiovascular therapies has remained very limited in the past decades compared to other clinical areas. Crucially, there is great difficulty in translating preclinical findings into safe and effective therapies: the probability of launch of a cardiovascular drug entering phase I is currently estimated at 4%, one of the lowest among all disease categories. In addition, cardiotoxicity is one of the most common adverse effects across multiple drug classes and an important cause of withdrawal from clinical trials or the market. One root cause is that commonly used preclinical models do not faithfully recapitulate human (patho)physiology. Most studies of cardiac development, function, and disease are based on animal models and particularly mice, but there are many confounding morphological and functional differences between the hearts of humans and rodents. Large animals such as pigs and non-human primates are better suited to translational research, but their use is limited by high costs of handling and ethical considerations. Conversely, classical *in vitro* cell-based models are amenable to high-throughput testing but suffer from low predictive value.

The epicardium is the mesothelial cell sheet covering the heart's outer surface. Long considered a simple barrier between the pericardial cavity and the myocardium, it is now recognized to hold key functions in cardiac development and repair. During embryonic development, a subset of epicardial cells undergoes epithelial-to-mesenchymal transition (EMT) to become epicardial derived cells (EPDCs) that migrate into the myocardium and give rise to the majority of fibroblasts and vascular smooth muscle cells of the heart. Whether EPDCs also differentiate into cardiomyocytes and coronary endothelial cells is still debated, with studies providing conflicting evidence. In addition to these cellular contributions, the epicardium provides signaling factors critical for the development and growth of the myocardium. It also plays a central role in heart regeneration in species capable of rebuilding adult heart muscle upon injury such as the zebrafish, making it a highly promising target for therapy. However, the inaccessibility of human embryonic tissue at the early stages of epicardium development, which begins less than four weeks post-conception, has left significant gaps in our understanding of human epicardial development and function. Many questions on the ontogeny of human proepicardial precursors and the functional heterogeneity of epicardial cells are still unresolved, which limits harnessing their full potential for regenerative medicine.

Human pluripotent stem cells (hPSCs) have emerged as a powerful alternative to generate *in vitro* models of higher physiological relevance. This includes embryonic stem cells (ESCs) and so-called induced pluripotent stem cells (iPSCs) that can be reprogrammed from the somatic cells of any healthy or diseased individual. The present inventors were the first to show that cardiac muscle cells (cardiomyocytes) differentiated from the iPSCs of a patient with long-QT syndrome type I recapitulated functional features of the disease (Moretti et al. (2010), "Patient-specific induced pluripotent stem-cell models for long-QT syndrome", N. Engl. J. Med.; 363:1397-1309). Importantly, pluripotent stem cells have the potential to form organoids, which are defined as self-organized 3D micro-tissues resembling an organ of the body in terms of cell composition, architecture, and function. In contrast to traditional tissue engineering, the generation of organoids relies on the self-patterning of cells upon modulation of signaling pathways that have been found to control the development of the organ *in vivo.* Organoids of most organs including the intestine, brain, kidney, lung, liver, and retina have already become versatile platforms for disease modeling, drug discovery, and personalized therapy. A notable exception is the heart: although the term organoid has been used in the literature to describe various 3D cardiac structures, no *bona fide* self-organized cardiac organoid composed of the three layers of epicardium, myocardium, and endocardium has yet been reported.

The present invention provides novel human pluripotent stem cell-derived cardiac micro-tissues showing self-organization of the myocardial and epicardial layers, which were named 'epicardioids'. Epicardioids recapitulate key steps of cardiac development and display the morphological and functional self-patterning typical of the ventricular wall. In addition, the inventors could show that the herein described epicardioids represent an advanced system to model multicellular mechanisms of heart disease.

### SUMMARY OF THE INVENTION

In one aspect, the invention relates to a heart tissue model named epicardioid comprising an inner myocardial compartment and an outer epicardial compartment, wherein the outer epicardial compartment comprises from the outside to the inside: a layer of epicardium comprising KRT18⁺ mesothelial epicardial cells , and a layer of subepicardium comprising Vim⁺ epcardial-derived cells (EPDCs); and wherein the inner myocardial compartment comprises from the outside to the inside: a layer of compact-like myocardium comprising densely packed cTNT⁺ myocardial cells and a layer of trabecular-like myocardium comprising looser packed cTnT⁺ myocardial cells compared to the compact-like myocardium, wherein the epicardioid has a three-dimensional shape, wherein the myocardial compartment forms the inner core of the epicardioid, wherein the epicardial compartment forms an envelope of the epicardioid, and wherein the epicardioid has been developed from one initial *in vitro* cell population.

In an embodiment, the initial *in vitro* cell population is an *in vitro* pluripotent stem cell population provided in cell culture medium, wherein the pluripotent stem cell population is preferably an induced pluripotent stem cell population, more preferably a human induced pluripotent stem cell population, or wherein the pluripotent stem cell population is preferably an embryonic stem cell population, more preferably a human embryonic stem cell population.

The cells of the outer epicardial compartment may further express epicardial transcription factor TBX18.

The layer of compacted myocardium may comprise a significantly higher proportion of Ki67⁺ proliferating cardiomyocytes than the inner trabecular-like layer of myocardium.

The inner myocardial compartment may further comprise in both layers, i.e., the compact-like and the trabecular-like myocardium, non-cardiomyocyte cells comprising cardiac fibroblasts, vascular smooth muscle cells and endothelial cells.

In an embodiment, the epicardioid matches with its two compartment structure the multilayered structure of the ventricular epicardium and myocardium of human embryos.

In another aspect, the invention relates to the use of the inventive epicadioid as a heart tissue model.

The herein disclosed epicardioid may be used for studying cardiac diseases, preferably left ventricular heart diseases.

The herein disclosed epicardioid may be used for preclinical testing of agents, compounds, drugs or other substances for the treatment of heart diseases. Such heart diseases are preferably hypertrophic and/or fibrotic heart diseases.

The herein disclosed epicardioid may also be used for preclinical testing of drug-induced arrhythmias.

In a further aspect, the herein disclosed epicardioid is provided for use in the treatment of cardiac diseases.

Such treatment may comprise the replacement of cardiomyocytes lost during myocardial infarction with the epicardioid, wherein the replacement is either by re-activating the epicardium's capacity to promote cardiomyocyte proliferation or by triggering *de novo* differentiation of EPDCs into cardiomyocytes.

In another aspect, the present invention relates to a method of producing a three-dimensional heart tissue model comprising an inner myocardial compartment and an outer epicardial compartment, wherein the outer epicardial compartment comprises from the outside to the inside a layer of epicardium comprising KRT18⁺ mesothelial epicardial cells, and a layer of subepicardium comprising Vim⁺ epcardial-derived cells (EPDCs); and wherein the inner myocardial compartment comprises from the outside to the inside a layer of compact-like myocardium comprising densely packed cTNT⁺ myocardial cells, and a layer of trabecular-like myocardium comprising looser packed cTnT⁺ myocardial cells compared to the compact-like myocardium, wherein the epicardioid has a three-dimensional shape, wherein the myocardial compartment forms the inner core of the epicardioid, and wherein the epicardial compartment forms the envelope of the epicardioid. The method of producing a three-dimensional heart issue model comprises the steps of: providing pluripotent stem cells (PCSs) and seeding the PSCs in an initial culture medium on day -1 of the culture; replacing the culture medium with a first basal differentiation medium on day 0 of the culture; replacing the culture medium with a second basal differentiation medium comprising retinoic acid on day 2 of the culture; refreshing the culture medium with the second basal differentiation medium each 24 hours until day 6 of the culture; replacing the culture medium with a third basal differentiation medium on day 6 of the culture; refreshing the culture medium with the third basal differentiation medium on day 7 of the culture; embedding spheroids into a gel comprising collagen type I, covering of the gel with a collagen type I solution, letting the collagen type I solution solidify, and transferring gel sheets to maintenance medium on day 8 of the culture; optionally placing the gel sheets on a rocking shaker on day 10 of the culture; replacing maintenance medium every 2-3 days after day 8 of the culture; isolating epicardioids for further use between days 13 and 100 of the culture, preferably between days 15 and 40.

The three-dimensional heart tissue model prepared by this method is an epcardioid as disclosed herein.

The PSCs are preferably human pluripotent stem cells, preferably human induced pluripotent stem cells (hiPSCs). Alternatively, the PSCs may be embryonic stem cells, preferably human embryonic stem cells.

In a preferred embodiment, the initial number of PSCs on day -1 of the culture is 30,000-40,000 per well of a 96-well plate.

The retinoic acid in the second basal differentiation medium is preferably present in a amount of 0.3-0.75 µM, preferably 0,5 µM.

In one embodiment, the initial culture medium comprises ROCK inhibitor thiazovivin. In a preferred embodiment, the initial culture medium is Essential 8 medium comprising 1-5 µM, preferably 2 µM thiazovivin.

In addition, the first basal medium preferably comprises BMP4, Activin A, bFGF, a PI3 kinase inhibitor, preferably LY-29004, and a Wnt activator, preferably 1 CHIR-99021.

The second basal medium preferably further comprises insulin, BMP4, bFGF, and a Wntantagonist, preferably IWP2.

The third basal medium preferably comprises insulin, BMP4, bFGF.

The collagen I solution comprises collagen I. In addition, the collagen I solution preferably comprises distilled water, DPBS, NaOH DMEM/F-12 with FBS, non-essential amino acids, and optionally Penicillin-Streptomycin.

In an embodiment, the maintenance medium comprises VEGF.

In a further embodiment, the 96-well plate comprises U-shaped wells which are optionally coated with a low cell adherence agent, preferably with poly-HEMA.

In another aspect, the invention relates to a method for screening or testing a candidate compound for its effects on heart development and/or functionality comprising producing a heart tissue model according to the method disclosed herein, while treating the cells with the candidate compound and comparing development of the heart tissue model with development and/or functionality of a heart tissue model that was not treated with the candidate compound.

In an embodiment, the compound to be tested is added to the culture during culture days 1 to 13. In another embodiment, the compound to be tested is added on or after day 13. In an embodiment, the compound is added once, in another embodiment, the compound is added more than once, for instance, each day, each second day or each third day of the culture.

In another aspect, the present invention further relates to a heart tissue model prepared according to the herein disclosed methods. In other words, the present invention further relates to a heart tissue model obtainable by the inventive methods described herein. The heart tissue model is preferably an epicardioid having the structure described herein.

The heart tissue model prepared according to the herein disclosed methods preferably comprises an inner myocardial compartment and an outer epicardial compartment, wherein the outer epicardial compartment comprises from the outside to the inside a layer of epicardium comprising KRT18⁺ mesothelial epicardial cells, and a layer of subepicardium comprising Vim⁺ epcardial-derived cells (EPDCs); and wherein the inner myocardial compartment comprises from the outside to the inside a layer of compact-like myocardium comprising densely packed cTNT⁺ myocardial cells, and a layer of trabecular-like myocardium comprising looser packed cTnT⁺ myocardial cells compared to the compact-like myocardium, wherein the epicardioid has a three-dimensional shape, wherein the myocardial compartment forms the inner core of the epicardioid and wherein the epicardial compartment forms the envelope of the epicardioid.

In an embodiment, the heart tissue model has been isolated from culture before day 13 of the culture, for instance at day 1, 2, 3, 4, 5, 6, 7, 8, 9, 10, 11, or 12 of the culture.

### ADVANTAGES OF THE INVENTION

In contrast to engineered heart tissues obtained by co-culture of individually differentiated (and/or native) cardiac cells as described in the prior art (Tyser et al., Science, 371, eabb2986 2021; Smart et al., Nature, 445:177-182 (2007); Moretti et al., Cell, 127:1151-1165 (2006)), the formation of epicardioids is driven by molecular processes that mimic embryonic cardiogenesis. This leads to tissue microarchitecture that more closely resembles *in vivo* organization, which is of advantage when modeling complex mechanisms of development or disease that depend on the interplay of form and function. Other groups have also generated cardiac microtissues from hPSCs based on developmental principles. Most notably, Hofbauer and colleagues recently generated human pluripotent stem cell-derived 'cardioids' consisting of myocardium forming an inner cavity lined with an endocardium-like layer (subject of patent application EP20164637.9). Drakhlis *et al.* reported the co-derivation of a cardiac structure - including a myocardial layer and an endocardium-like compartment - with foregut endoderm (Drakhlis et al., (2021), "Human heart-forming organoids recapitulate early heart and foregut development"; Nat. Biotehcnol.; 396(39):737-746). Lewis-Israeli *et al.* generated cardiac structures containing endothelial cells as well as epicardial cells forming individual clusters (Lewis-Israeli et al., (2021); "Self-assembling human heart organoids for the modeling of cardiac development and congenital heart disease"; Nat. Commun., 12:5142). Others reported *in vitro* development of early cardiac structures corresponding to the early crescent stage (Rossi et al., (2021); "Capturing Cardiogenesis in Gastruloids"; Cell Stem Cell, 28:230-240.e6; Andersen et al., (2018); Precardiac organoids from two heart fields via Bmp/Wnt signaling"; Nat. Commun., 9:3140)).

However, to our knowledge, herein described epicardioids are the first to show spontaneous organization and maintenance of a continuous epicardial layer as well as an advanced morphological and functional self-patterning of the myocardial layer. The disclosed invention provides for the possibility to established human epicardioids showing retinoic acid-dependent self-organization of ventricular myocardium and epicardium. Epicardioids recapitulate the two major functions of the embryonic epicardium: being the source of progenitors of several cardiac lineages and providing a paracrine milieu driving myocardial compaction and maturation. The latter appears to be key in achieving a high degree of morphological, molecular, and functional self-patterning of the myocardium, which has so far been lacking in cardiac organoid models.

Single-cell transcriptomic analyses of human embryonic and adult heart tissue have provided precious insights into epicardial development. However, isolated tissues represent punctual snapshots that are of limited use for studying dynamic developmental processes, especially those occurring at the earliest stages of cardiogenesis. The epicardioids of the present invention offer a powerful alternative, as they closely mimic the steps of left ventricular development and maturation.

During their *in vitro* development, the disclosed heart tissue models notably revealed the existence of a human equivalent of the murine juxta-cardiac field (JCF) showing uni- or bipotentiality for myocardial and epicardial fates associated with distinct gene regulatory programs. ISL1 was identified herein as a marker of the human JCF and early embryonic epicardium.

The disclosed heart tissue models, especially the epicardioids of the invention can be used to address open questions related to epicardial fate potential. For instance, combined transcriptomic and chromatin accessibility profiling supported the still debated myocytic potential of early epicardium, though with the epicardioids of the invention evidence of a decline of this potential at the chromatin level over time could be gained. Importantly, for all three epicardial derivatives - fibroblasts, smooth muscle cells, and cardiomyocytes - dynamic changes in fate potential rather than strict pre-determination was observed. This is consistent with recent work in the mouse challenging the long-held notion that there exist distinct epicardial subcompartments.

Finally, the invention demonstrates that mature epicardioids have the unique ability of recapitulating both hypertrophic and fibrotic features of left ventricular hypertrophy. They could therefore be exploited for preclinical testing to identify drugs targeting both aspects of the disease, which are intimately linked during the progression towards heart failure. More broadly, the epicardioids of the invention offer advantages for modeling complex cardiac disorders including congenital heart diseases by allowing dissection of inter- and intra-cellular crosstalk dynamics during disease development.

Insights from epicardioids also lead to new strategies to replace cardiomyocytes lost during myocardial infarction, arguably one of the biggest challenges of modern medicine, either by re-activating the epicardium's capacity to promote cardiomyocyte proliferation or by triggering de *novo* differentiation of EPDCs into cardiomyocytes.

As such, the disclosed heart tissue models, especially the disclosed epicardioids, offer a unique platform to tackle fundamental questions in developmental biology as well as cardiovascular medicine and drug discovery.

### DESCRIPTION OF THE FIGURES

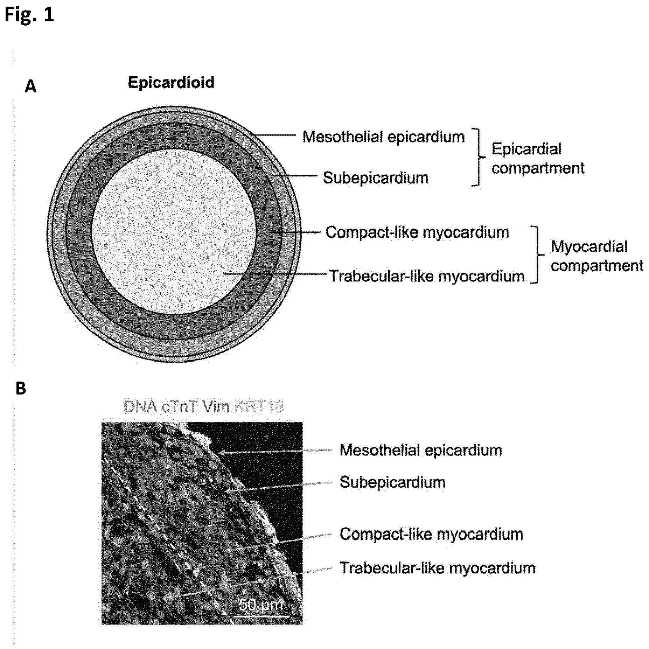

**Figure 1****:** A Schematic drawing of an epicardioid. Shown are the different compartments of the epicardioid, each having characteristic layers of cells. **B** Confocal microscopy picture showing the epicardioid structure based on different cell stains. KRT18: mesothelial epicardial cells, Vim: subepicardial EPDCs, cTnT: myocardial cells.

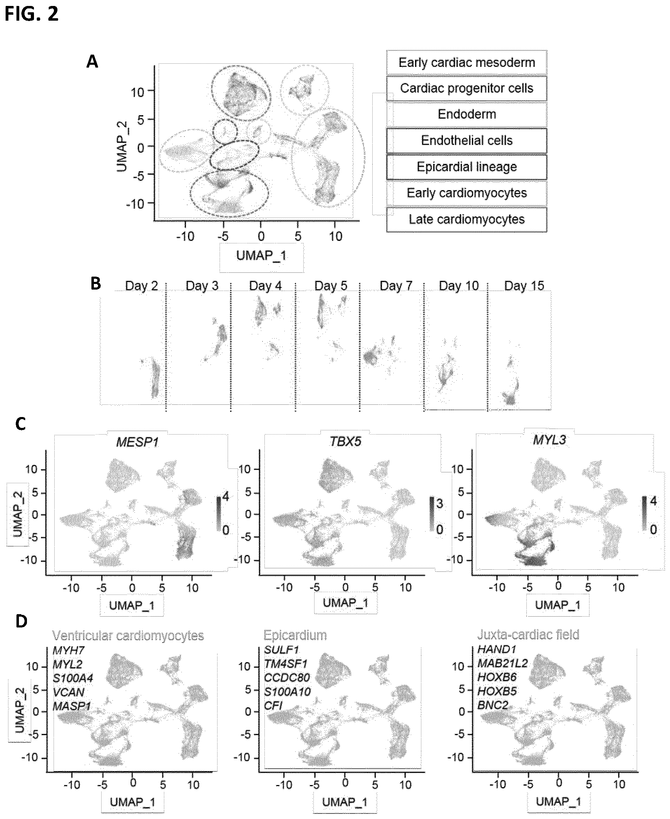

**Figure 2****:** Time course single-cell RNA sequencing analysis reveals molecular processes of epicardioid formation. (**A**,**B**). Epicardioids were analyzed by single-cell RNA-sequencing (10× Genomics) at days 2, 3, 4, 5, 7, 10, and 15 of culture. The 31 clusters identified across all samples are broadly annotated (A) and separated by day (B). (C) UMAP feature plots of MESP1, TBX5 and MYL3 in epicardioids across all time points. Heatmaps indicate the respective level of expression of each gene. (D) UMAP feature plot showing cells co-expressing the indicated markers of human ventricular cardiomyocytes, human epicardium, and murine juxta-cardiac field in epicardioids across all time points.

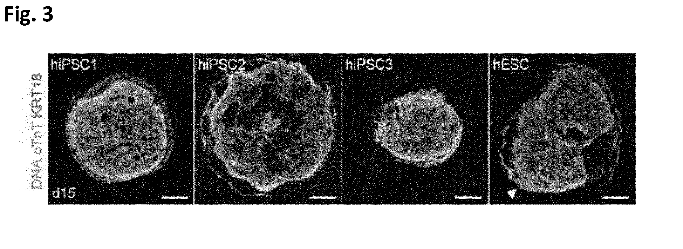

**Figure 3****:** Representative images of cTnT and cytokeratin 18 (KRT18) immunostaining of epicardioids differentiated from four different hPSC lines (3 hiPSC lines and 1 hESC line) at day 15 of culture. The arrowhead indicates an example of a partial epicardial layer.

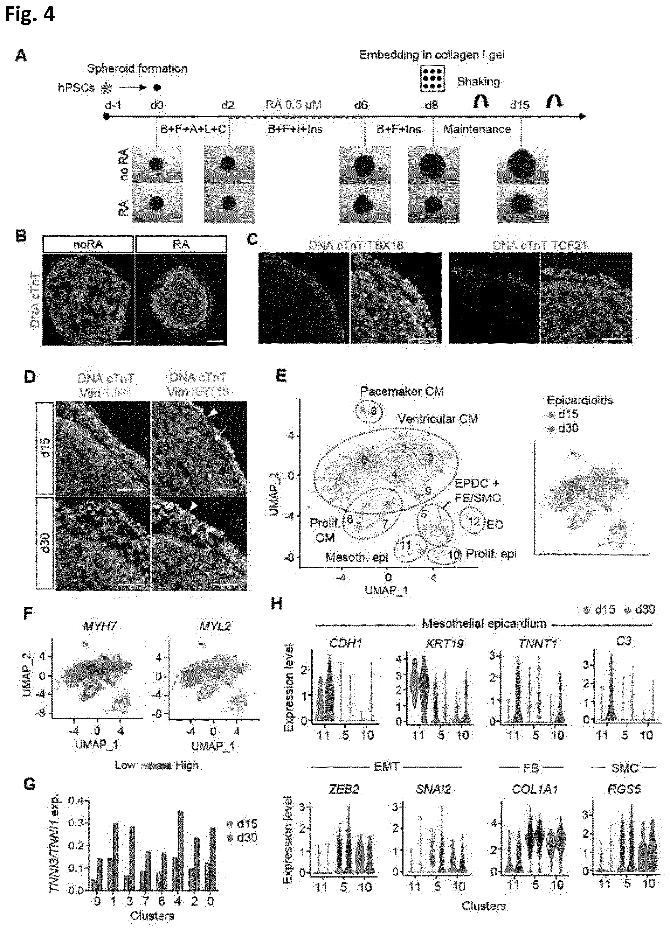

**Figure 4****:** Generation of human pluripotent stem cell-derived epicardioids showing selforganized myocardium and epicardium. **A**: Protocol used for 3D cardiac induction of hPSCs,with addition of retinoic acid (RA) or without and representative bright field images at the indicated days. B: BMP4, F: FGF2, A: Activin A, L: LY-29004, C: CHIR-99021, I: IWP2, Ins: Insulin. Scale bars = 500 µm. **B**,**C**: Representative images of immunostaining for the cardiomyocyte (CM) marker cardiac troponin T (cTnT) in spheroids differentiated with RA or without (noRA) (**B**) and immunostaining for cTnT and the epicardial markers TBX18 and TCF21 in spheroids differentiated with RA (**C**), all at day 15 of culture. Scale bars B = 200 µm, C = 50 µm. **D**: Representative images of immunostaining for cTnT, vimentin (Vim), and the epithelial markers cytokeratin 18 (KRT18) and tight junction protein 1 (TJP1) in spheroids differentiated with RA, referred to hereafter as epicardioids, at days 15 and 30 of culture. Arrowheads indicate the mesothelial epicardium, arrows indicate subjacent EPDCs. Scale bars = 50 µm. **E**: (Left) UMAP dimensional reduction plot showing the cell clusters obtained by single-cell RNA sequencing of epicardioids at days 15 and d30, with main cell types annotated (except for 15 unclassified clusters 13 and 14). (Right) Cells from day 15 and day 30 are highlighted in orange and blue, respectively. CM: cardiomyocytes, EPDC: epicardium-derived cells, FB: fibroblasts, SMC: smooth muscle cells, EC: endothelial cells, prolif.: proliferative, mesoth.: mesothelial, epi: epicardial cells. F: Feature plots showing the expression level of ventricular CM markers (MYH7, MYL2). G: Average expression ratio of the adult to fetal cardiac troponin I isoforms 20 (TNNI3/TNNI1) in the indicated ventricular CM clusters at days 15 and 30 of culture. H: Violin plots showing the expression levels of markers of mesothelial epicardium (CDH1, KRT19, TNNT1, C3), EMT (ZEB2, SNAI2), fibroblasts (FB; COL1A1), and smooth muscle cells (SMC; RGS5) in the indicated epicardial clusters at days 15 and 30 of culture.

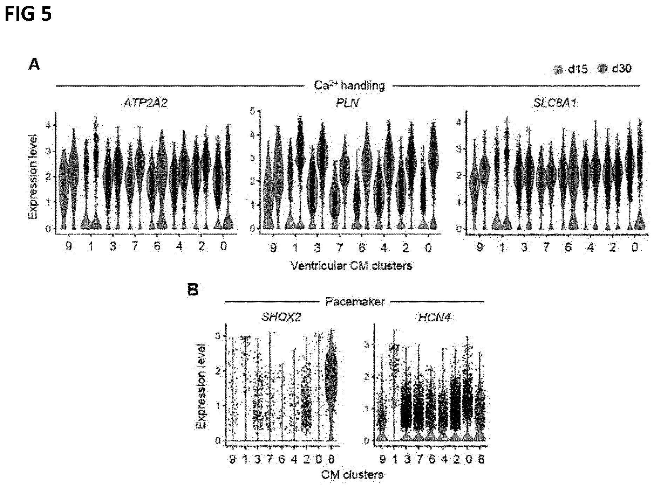

**Figure 5****:** Transcriptional profiles of cardiomyocytes in epicardioids at days 15 and 30. A: Violin blots of the expression levels of genes related to cardiomyocyte (CM) Ca2+ handling (ATP2A2, PLN, SLC8A1) in the indicated ventricular CM clusters identified by scRNA-seq. Cells from epicardioids at day 15 and 30 are shown separately. **B:** Violin plots of the expression levels of markers of pacemaker CMs (SHOX2, HCN4) in the indicated scRNA-seq clusters.

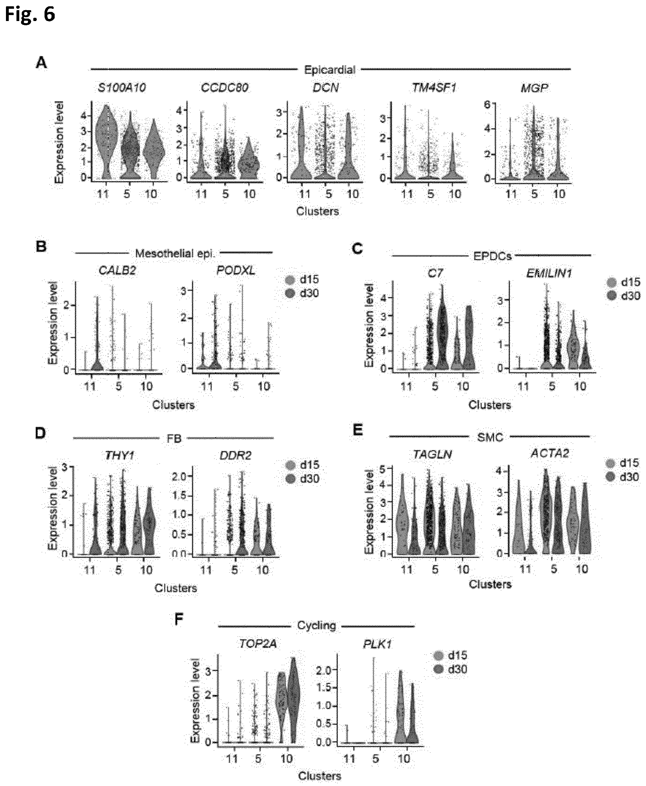

**Figure 6****:** Transcriptional profiles of epicardial cells and their derivatives in epicardioids at days 15 and 30. A: Violin plots of the expression levels of human epicardial genes (S100A10, CCDC80, DCN, TM4SF1, MGP) in the indicated scRNA-seq clusters. **B-F**: Violin plots of the expression levels of markers of mesothelial epicardium (CALB2, PODXL) (B), epicardium-derived cells (EPDCs; C7, EMILIN1) (C), fibroblasts (FB; THY1, DDR2) (D), smooth muscle cells (SMC; TAGLN, ACTA2) (E), and mitotic cells (TOP2A, PLK1) (F) in the indicated scRNA-seq clusters. Cells from epicardioids at day 15 and 30 are shown separately.

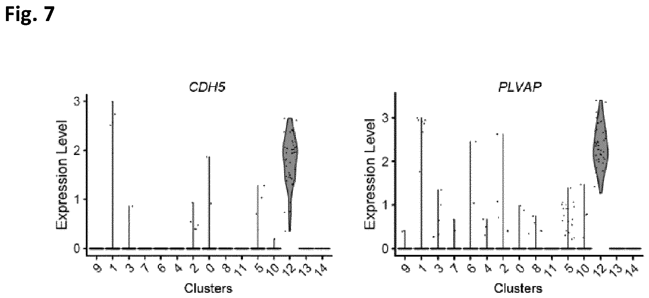

**Figure 7****:** Endothelial cells in epicardioids at days 15 and 30. Violin plots of the expression levels of endothelial cell markers (CDH5, PLVAP) in the indicated scRNA-seq clusters.

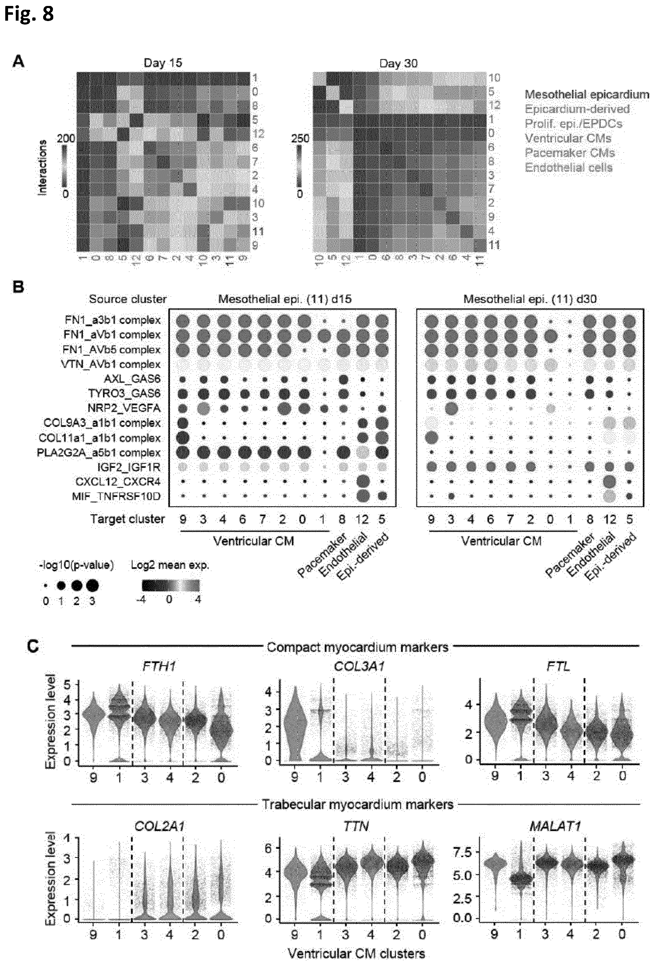

**Figure 8****:** Epicardioids recapitulate the cellular crosstalk promoting ventricular patterning. A: Heatmap showing the number of cell-cell interactions between all scRNA-seq clusters in epicardioids at day 15 and 30 of culture. Main cell types are annotated, unclassified clusters (13, 14) were excluded. **B**: Dot plot showing selected ligand-receptor interactions between mesothelial epicardium (cluster 11) and the indicated cell clusters in epicardioids at day 15 and 30. P-values are indicated by circle size, the mean of the expression level of the two interacting molecules in the respective clusters are indicated by color. C:Violin plots showing the expression levels of markers of human compact (FTH1, FTL, COL3A1) and trabecular (COL2A1, TTN, MALAT1) myocardium in the indicated ventricular cardiomyocyte (CM) clusters. CMs clustering based on high cell cycle activity rather than identity (clusters 6 and 7) were excluded. Dashed lines show the distinction between clusters displaying compact (9, 1), trabecular (2, 0), and intermediate (3, 4) transcriptional profiles.

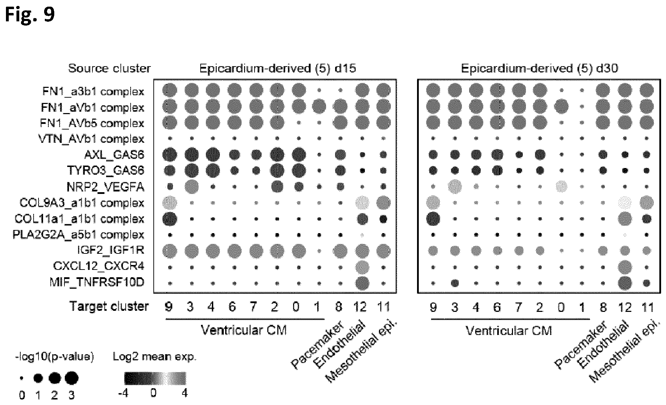

**Figure 9****:** Cell-cell communication in epicardioids. Dot plot showing ligand-receptor interactions between epicardium-derived cells (cluster 5) and the indicated cell clusters in epicardioids at day 15 and 30. Pvalues are indicated by circle size, the mean of the expression level of the two interacting molecules in the respective clusters are indicated by color.

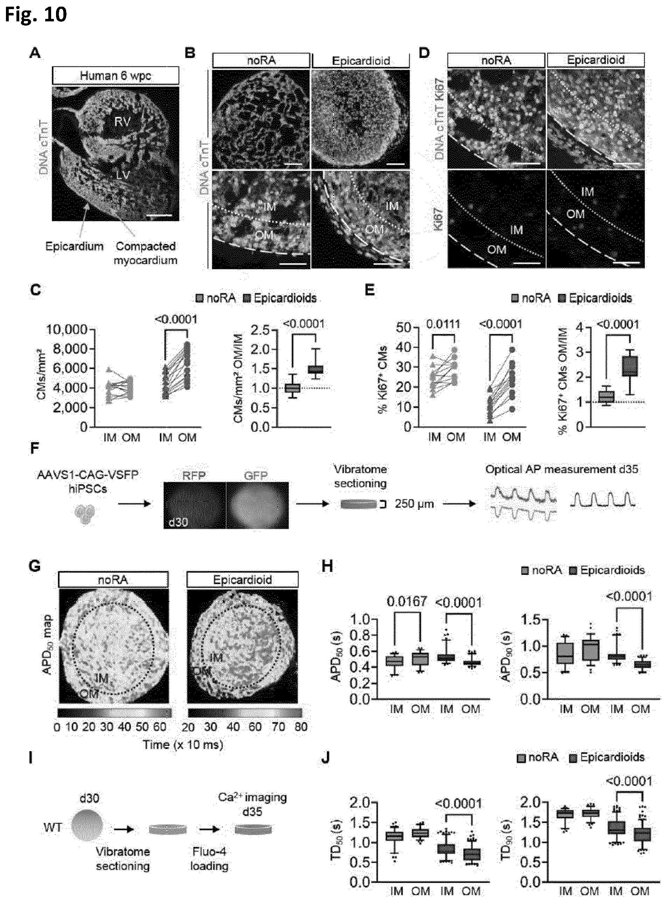

**Figure 10****:** Epicardioids display morphological and functional self-patterning of the myocardium. A,B: Representative images of immunostaining for cardiac troponin T (cTnT) in human ventricular tissue at 6 weeks post conception (wpc) (A) and in noRA spheroids and epicardioids at day 15 (B). The hatched lines mark the outer edge of the myocardium; the dotted lines mark the delimitation between the outer myocardium (OM, defined as the myocardium within 50 µm of the outer edge across all samples) and the inner myocardium (IM). Scale bars in (A) = 500 µm, (B) top = 100 µm, bottom = 50 µm. C: (Left) Density of cardiomyocytes (CMs) in the OM and IM of noRA spheroids and epicardioids at day 15. Dot plots showing all data points; lines connect the values for OM and IM within the same sample. N = 15 per group; 2-way ANOVA matching paired data with Sidak's multiple comparison test. (Right) Corresponding ratio of CM density between OM and IM; box plots indicate the median, 25th and 75th percentile, with whiskers extending to the 5th and 95th percentiles. N = 15 per group; unpaired two-tailed t-test. D: Representative images of immunostaining for cTnT and the cell cycle activity marker Ki67 in noRA spheroids and epicardioids at day 15. Scale bars = 50 µm. E: (Left) Percentage of Ki67+ 15 CMs in the OM and IM of noRA spheroids and epicardioids at day 15. Dot plots showing all data points; lines connect the values for OM and IM within the same sample. N = 12 per group; 2-way ANOVA matching data with Sidak's multiple comparison test. (Right) Corresponding ratio of the percentage of Ki67+ CMs between OM and IM; box plots indicate the median, 25th and 75^{th} percentile, with whiskers extending to the 5th and 95th percentiles. N = 12 per group; unpaired two tailed t-test. F: Graphical representation of the 3D differentiation of hiPSCs expressing a FRETbased voltage indicator (AAVS1-CAG-VSFP) followed by vibratome cutting of 250 µm-thick slices at day 30 for optical action potential (AP) measurement at day 35. G: Representative map of action potential duration to 50% repolarization (APD50) in a noRA spheroid and epicardioid at day 35. **H**: Quantification of APD50 (left) and APD90 (right) in the OM and IM of noRA spheroids and epicardioids under 0.5 Hz pacing at day 35. Box plots indicate the median, 25th and 75^{th} percentile, with whiskers extending to the 5th and 95th percentiles. N = 60 APs per layer from 3 noRA spheroids; N = 100 APs per layer from 6 epicardioids; Kruskal-Wallis test with Dunn's multiple comparison test. **I**: Graphical representation of Ca2+ transient imaging in 250 µm-thick slices cut by vibratome at day 30 and loaded with the fluorescent C 5 a2+ indicator Fluo-4 at day 35. J: Quantification of the time to 50% or 90% peak decay (TD50, left and TD90, right) in the OM and IM of noRA spheroids and epicardioids under 0.5 Hz pacing at day 35. Box plots indicate the median, 25th and 75th percentile, with whiskers extending to the 5th and 95th percentiles. N = 75 transients per layer from 4 noRA spheroids; N = 200 transients per layer from 9 epicardioids; Kruskal-Wallis test with Dunn's multiple comparison test.

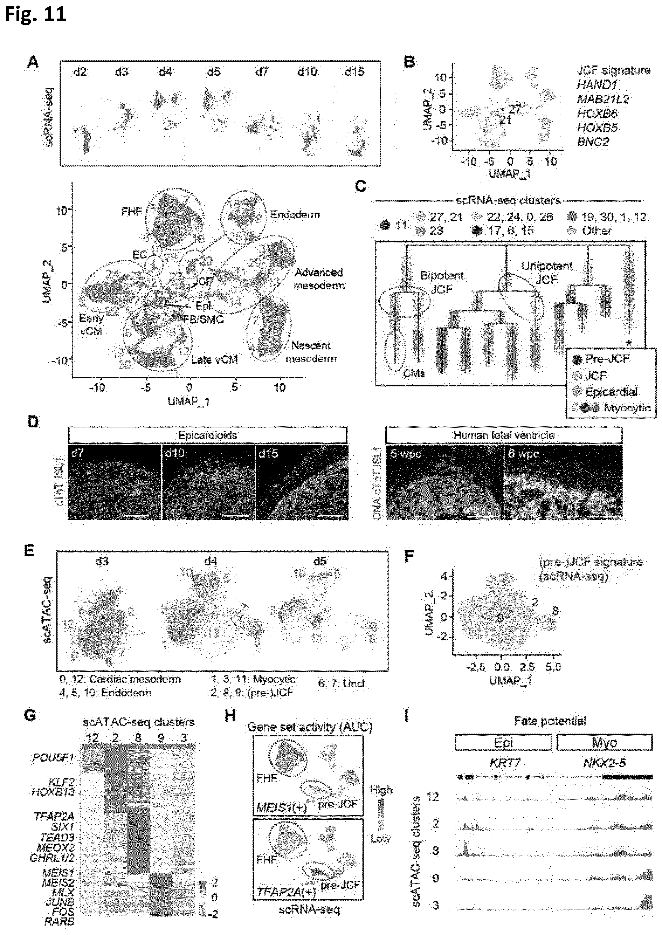

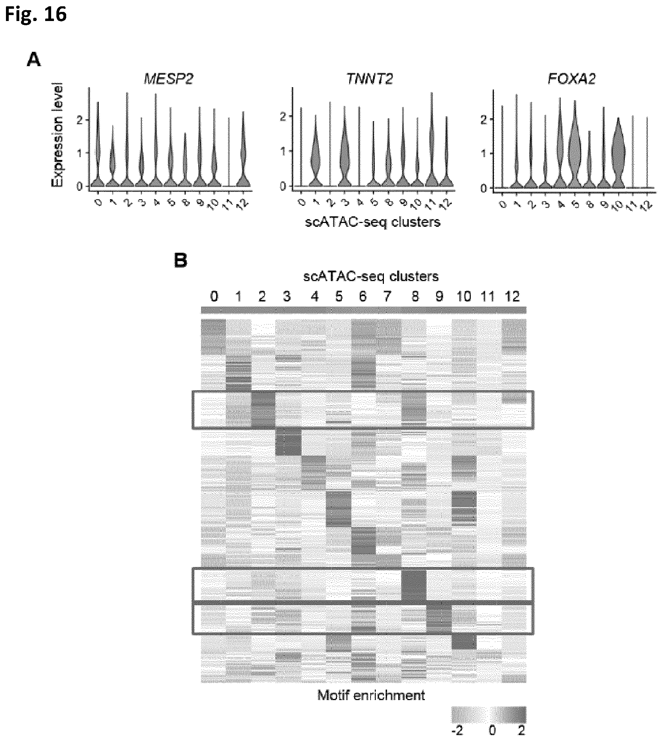

**Figure 11****:** Epicardioids are formed by early segregation of first heart field (FHF) and juxtacardiac field (JCF) progenitors. A: UMAP dimensional reduction plot of the 31 scRNA-seq clusters obtained based on gene expression in epicardioids on days 2, 3, 4, 5, 7, 10, and 15 of differentiation, separated by sample (top) and merged (bottom). Cluster numbers and main cell types are annotated. FHF: first heart field; JCF: juxta-5 cardiac field; EC: endothelial cells; vCM: ventricular cardiomyocytes; Epi: epicardium; FB: fibroblasts; SMC: smooth muscle cells. **B**: UMAP plot showing cells co-expressing markers of the juxta-cardiac field (JCF; HAND1, MAB21L2, HOXB6, HOXB5, BNC2) in red. **C**:Computational reconstruction of the developmental trajectories of cells from day 2 to 15 by URD analysis. The indicated clusters corresponding to pre-JCF, JCF, epicardial, and myocytic lineages are highlighted. The asterisk indicates cells segregating based on high mitotic activity. Circles highlight putative unipotent and bipotent JCF populations as well as cardiomyocyte (CMs) appearing downstream of the epicardial lineage. **D**: Representative images of immunostaining for the cTnT and ISL1 in epicardioids at days 7, 10, and 15. (left) Representative images of immunostaining for cTnT and ISL1 in 15 ventricular tissue from human embryos at 5 and 6 weeks post conception (wpc) (right). Scale bars = 50 µm. **E**: UMAP visualization of cell clusters identified based on chromatin accessibility via scATAC-seq analysis of epicardioids at days 3, 4, and 5. Main cell types are annotated; uncl.: unclassified. **F**: UMAP visualization of cells matching the (pre-)JCF signature derived from scRNA-seq analysis (labelled in red) among the scATAC-seq clusters presented in D. Negative 20 cells are shown in grey. **G**: Heatmap showing average Z-scores of transcription factor motif accessibility across the indicated scATAC-seq clusters. Clusters and motifs relevant to epicardial and myocytic commitment were selected (full heatmap presented in Fig. 16B); defining transcription factors are highlighted. **H**: UMAP visualization of the gene set activity (AUC) of the MEIS1 and TFAP2A regulons among scRNA-seq clusters at days 3, 4, and 5. Dotted circles highlight FHF and pre-JCF clusters. **I**: Average coverage plots of KRT7 and NKX2.5 in the scATAC-seq clusters 12, 2, 8, 9, and 3, illustrating potential for epicardial and myocytic fates, respectively.

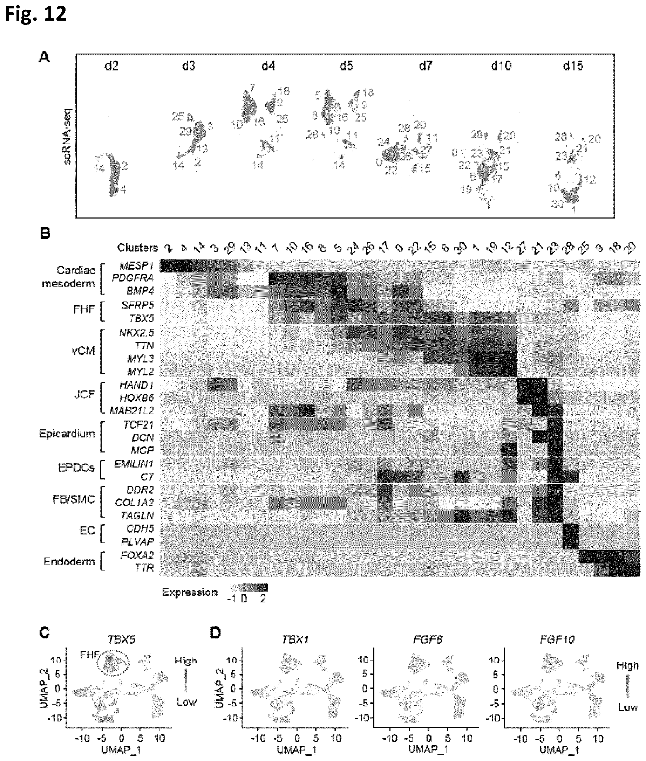

**Figure 12****:** Transcriptional dynamics during epicardioid development. **A**: UMAP plot showing the temporal distribution of the 31 clusters obtained by scRNA-seq analysis of epicardioids at days 2, 3, 4, 5, 7, 10, and 15 of differentiation. **B**: Heatmap showing the average expression of markers of the indicated cell types in all clusters. FHF: first heart field; vCM: ventricular cardiomyocytes; JCF: juxta-cardiac field; EPDCs: epicardium-derived cells; FB: fibroblasts; SMC: smooth muscle cells; EC: endothelial cells. C,D: UMAP plots showing the expression levels of markers of the FHF (TBX5) (C) and anterior SHF (TBX1, FGF8, FGF10) (D) across all time points.

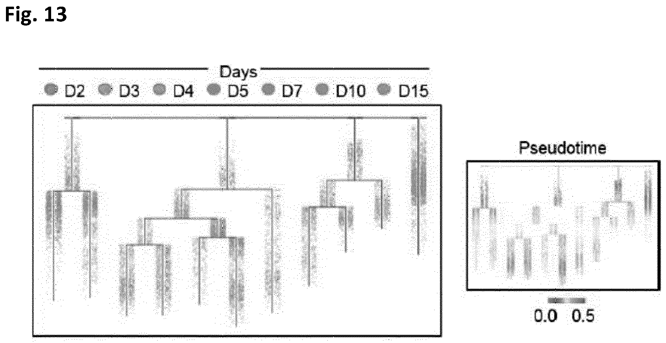

**Figure 13****:** Developmental trajectories of cells during epicardioid development. (Left) Computational reconstruction of the developmental trajectories of cells from day 2 to day 15 by URD analysis, with cells colored by day. (Right) Corresponding plot showing cells colored according to their relative developmental stage in pseudotime.

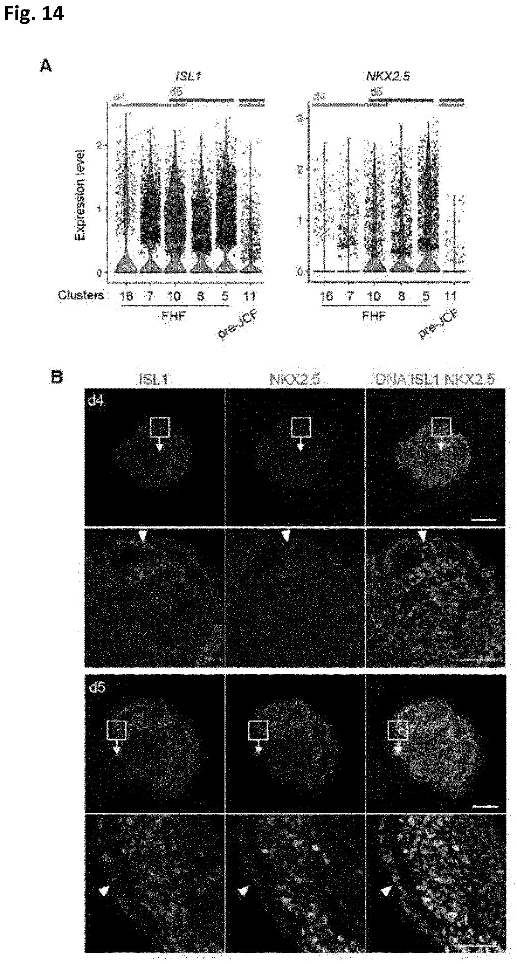

**Figure 14****:** Progenitors of the JCF and FHF show spatial segregation in early epicardioids. A: Violin plots showing the expression levels of ISL1 and NKX2.5 in the indicated FHF and pre-JCF scRNA-seq clusters, predominantly present at the indicated days. (B) Representative images of immunostaining for ISL1 and NKX2.5 in epicardioids at days 4 (top) and 5 (bottom). Arrowheads show examples of a layer of ISL1+/NKX2.5- JCF progenitors segregating from ISL1+/NKX2.5+ FHF progenitors. For each day scale bars top = 200 µm, bottom = 50 µm.

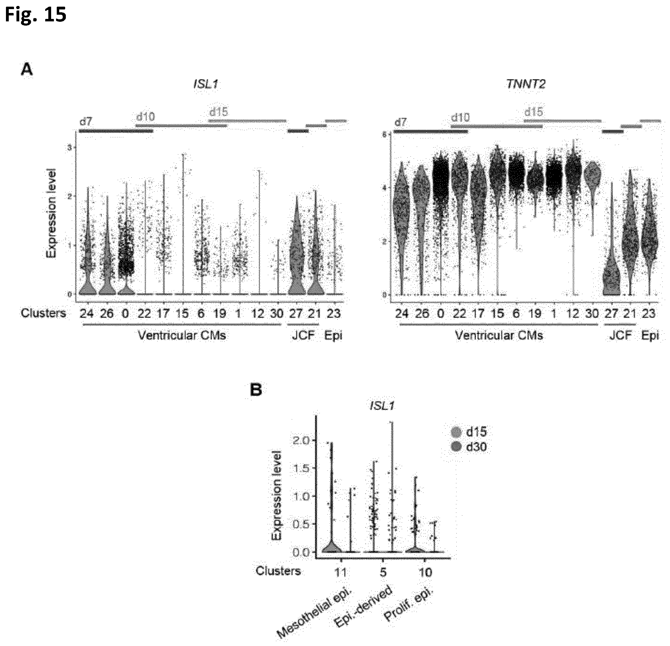

**Figure 15****:** ISL1 is a marker of the human JCF and is temporarily maintained in mesothelial epicardium. **A**: Violin plots showing the expression levels of ISL1 and TNNT2 in the indicated ventricular cardiomyocyte (CM), juxta-cardiac field (JCF) and epicardial (epi) clusters obtained from scRNA-seq analysis of epicardioids from day 2 to day 15; clusters are predominantly present at the indicated days. **B**: Violin plots of the expression level of ISL1 in the indicated clusters obtained from scRNA-seq analysis of epicardioids at day 15 and 30; cells from day 15 and 30 are shown separately.
**Figure 16****:** Chromatin accessibility profiling by scATAC-seq in early epicardioids. A: Violin plots showing the predicted expression of markers of cardiac mesoderm (MESP2), cardiomyocytes (TNNT2) and endoderm (FOXA2) in the 13 clusters obtained by unsupervised clustering analysis of scATAC-seq data from epicardioids at days 3, 4, and 5. **B**: Heatmap showing average Z-scores of transcription factor motif accessibility across the indicated scATAC-seq clusters. Motifs relevant to epicardial and myocytic commitment are framed in blue and presented in Fig. 11G for selected clusters.

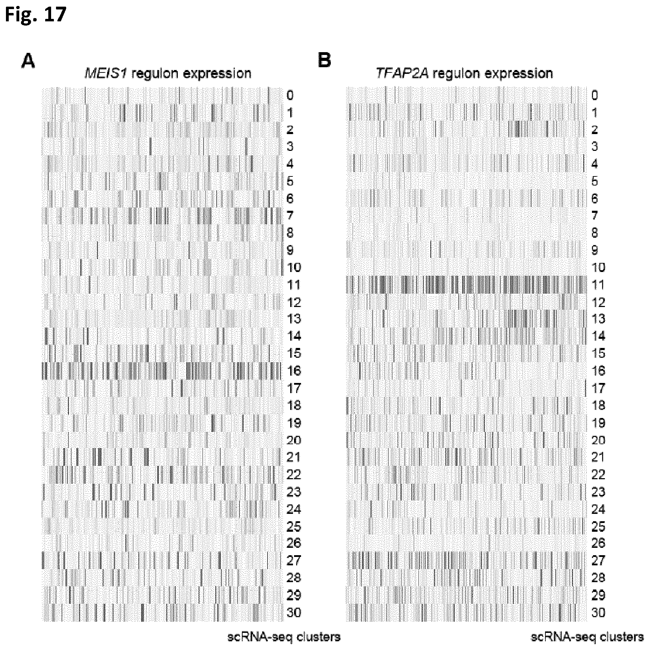

**Figure 17****:** Activity of the MEIS1 and TFAP2A regulons in early epicardioids. **A**,**B**: Heatmap showing the level of expression of members of the MEIS1 (A) and TFAP2A (B) regulons in all clusters obtained by scRNAseq analysis of epicardioids from days 2 to 15.

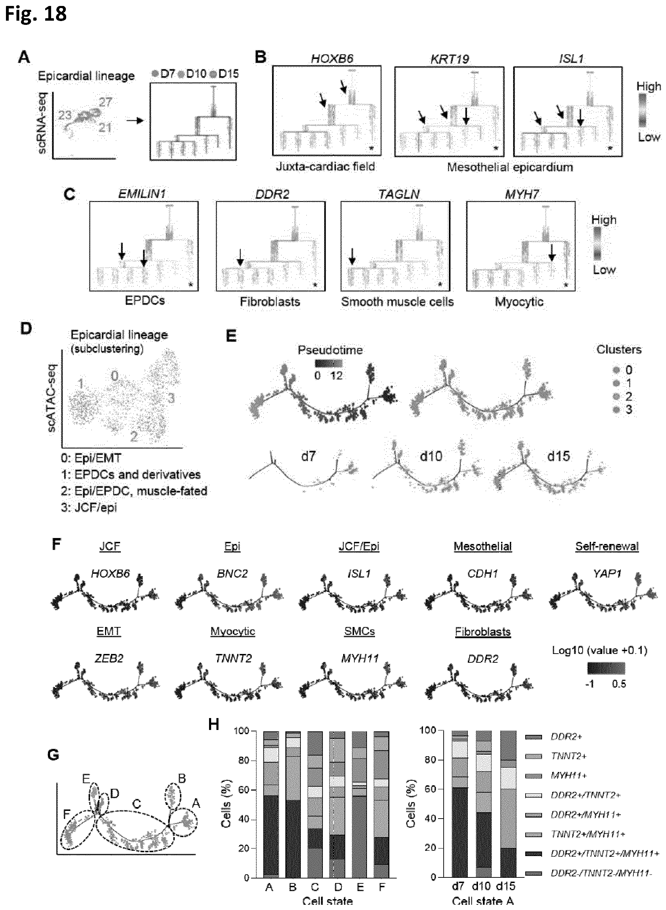

**Figure 18****:** Single-cell whole-transcriptome and chromatin accessibility analyses uncover transcriptional programs guiding fate decisions along the epicardial lineage tree. A: Computational reconstruction of the developmental trajectories of the JCF and epicardial scRNAseq clusters 27, 21, and 23 on days 7, 10, and 15 by URD analysis. B,C: URD plots showing the level of expression of the JCF marker HOXB6, 5 the mesothelial epicardium marker KRT19, ISL1, (C) the myocytic marker MYH7, the epicardium-derived cell (EPDC) marker EMILIN1, the fibroblast marker DDR2, and the smooth muscle marker TAGLN (D) along the trajectories presented in B. Arrows highlight branches with the indicated cell identities. The asterisk indicates cells segregating based on high mitotic activity. D: UMAP visualization of cell clusters identified based on chromatin accessibility after subclustering of the epicardial clusters 5 and 6 obtained via scATAC-seq analysis of epicardioids at days 7, 10, and 15. Main cell types are annotated. Epi: epicardium; EMT: epithelial-to-mesenchymal transition; EPDCs: epicardium-derived cells; JCF: juxta-cardiac field. E: Monocle-generated plot showing the pseudotime ordering and differentiation trajectories of the scATAC-seq clusters presented in (E). Top: cells are colored according to increasing maturation level from dark to light (left) or cluster identity (right). Bottom: plots are colored by cluster identity and split by time point. F: Monocle-generated plots showing the pseudotime-ordered inferred gene activity of markers of the indicated cell states (underlined). Shades of blue indicate reduced gene activity and shades of red indicate increased gene activity. JCF: juxta-cardiac field; epi: epicardium; EMT: epithelial-to-mesenchymal transition; EPDCs: epicardium-derived cells; SMCs: smooth muscle cells. G: Representation of cell states A-F along the pseudotrajectory based on the developmental progression of cells in the epicardial subclusters 0, 1, 2, 3 at days 7, 10, and 15. H: (Left) Percentage of cells showing inferred gene activity for the fibroblast marker DDR2, the smooth muscle cell marker MYH11, and the cardiomyocyte marker TNNT2 and the indicated combinations in each cell state defined in (G). (Right) Percentage of cells showing inferred gene activity for the fibroblast marker DDR2, the smooth muscle cell marker MYH11, the cardiomyocyte marker TNNT2 and the indicated combinations of genes in cell state A at days 7, 10, and 15.

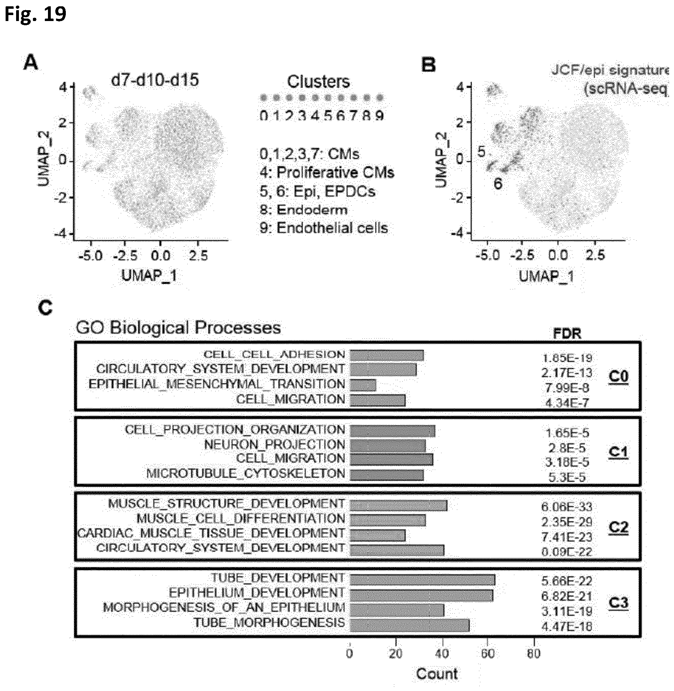

**Figure 19****:** Chromatin accessibility profiling of the epicardial lineage via single-cell ATAC-seq in epicardioids at days 7, 10, and 15. **A**: UMAP visualization of cell clusters identified based on chromatin accessibility via scATAC-seq analysis of epicardioids at days 7, 10, and 15. Main cell types are annotated. CMs: cardiomyocytes, Epi: epicardium, EPDCs: epicardium-derived cells. **B**: UMAP visualization of cells matching the epicardial signature derived from scRNA-seq analysis (labelled in red) among the scATACseq clusters presented in (A). Negative cells are shown in grey. (C) Bar chart of gene ontology (GO) categories identified by gene set enrichment analysis (GSEA) of clusters 0, 1, 2, and 3 obtained by subclustering of the epicardial scATAC.seq clusters 5 and 6 (FDR ≤ 0.001). Bars represent the number of genes present in each category.

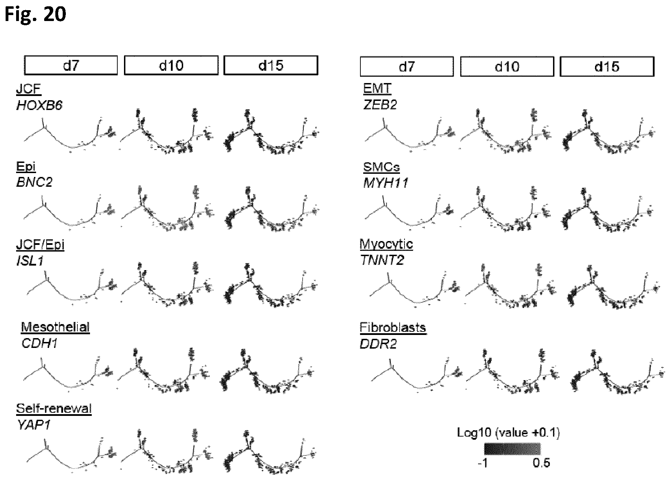

**Figure 20****:** Chromatin accessibility dynamics reveal the fate trajectories of epicardial cells. Monocle-generated plots showing the pseudotime-ordered inferred gene activity of markers of the indicated cell states (underlined) in the epicardial subclusters 0, 1, 2, 3 at days 7, 10, and 15. Shades of blue indicate reduced gene activity and shades of red indicate increased gene activity. JCF: juxta-cardiac field; epi: epicardium; EMT: epithelial-to-mesenchymal transition; EPDCs: epicardium-derived cells; SMCs: smooth muscle cells.

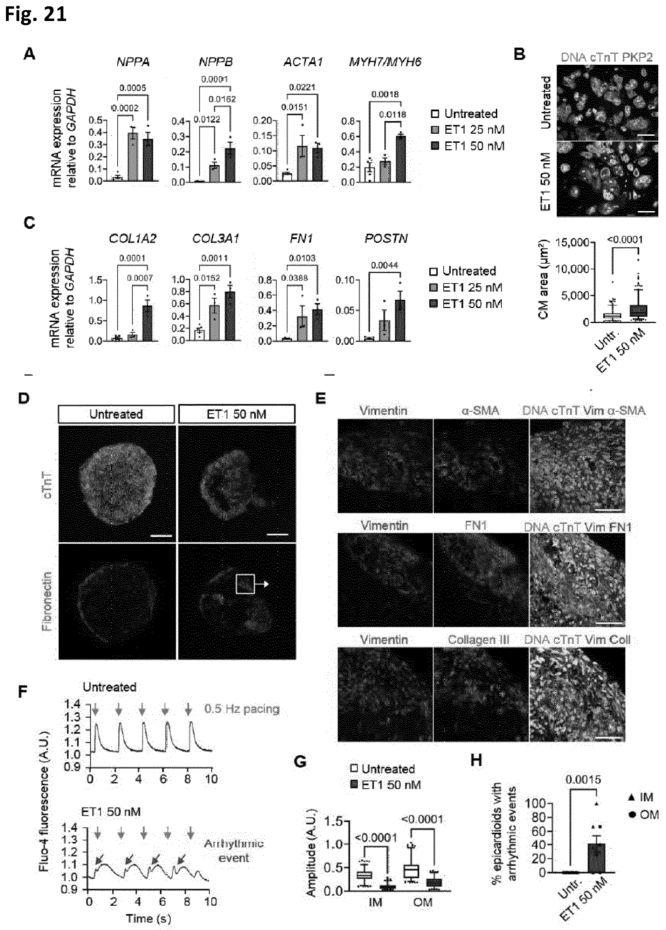

**Figure 21****:** Endothelin-1 stimulation triggers hypertrophic and fibrotic responses in epicardioids. A: mRNA expression of NPPA, NPPB, ACTA1, and MYH7/MYH6 ratio relative to GAPDH in epicardioids treated with 25 nM or 50 nM endothelin-1 (ET1) compared to untreated controls. Mean ± SEM; N = 3 per group; one-way ANOVA with Sidak's multiple comparison test. **B**: (Top) Representative images of cardiomyocytes (CMs) re-seeded after dissociation of untreated or ET1-treated epicardioids and stained for cardiac troponin T (cTnT) and the desmosomal marker plakophilin-2 denoting cell membrane (PKP2). Scale bars = 100 µm. (Bottom) Corresponding quantification of CM area, box plots indicate the median, 25th and 75th percentile, with whiskers extending to the 5th and 95th percentiles. N = 260 CMs from 3 differentiations per group; unpaired two-tailed t-test. C: mRNA expression of COL1A2, COL3A1, FN1, and POSTN relative to GAPDH in epicardioids treated with 25 nM or 50 nM ET1 compared to untreated controls. Mean ± SEM; N = 3 per group; one-way ANOVA with Sidak's multiple comparison test. **D**: Representative images of immunostaining for cTnT and fibronectin in untreated or ET1-treated epicardioids. Scale bars = 200 µm. The square highlights the outer layer of ET1-treated 15 epicardioids further analyzed in (E). E: Representative images of immunostaining for cTnT, α-smooth muscle actin (α-SMA), fibronectin (FN1), and collagen type III (Coll) in ET1-treated epicardioids. Scale bars = 50 µm. F: Exemplary traces of Fluo-4 fluorescence in untreated or ET1-treated epicardioids. Blue arrows indicate 0.5 Hz pacing, red arrows indicate examples of arrhythmic events. G: Amplitude of Ca2+ transients in the outer (OM) or inner myocardium (IM) 20 of untreated or ET1-treated epicardioids, box plots indicate the median, 25th and 75th percentile, with whiskers extending to the 5th and 95th percentiles. Untreated: N = 130 transients/layer from 5 epicardioids, ET1: N = 106 transients/layer from 4 epicardioids; Kruskal-Wallis test with Dunn's multiple comparison test. H: Percentage of untreated or ET1-treated epicardioids displaying arrhythmic events in the OM or IM. Mean ± SEM; untreated N = 5 epicardioids, ET1: N = 4 epicardioids; unpaired two-tailed t-test.

### DEFINITION OF TERMS

Unless otherwise defined, all technical and scientific terms used herein have the same meaning as commonly understood by one of ordinary skill in the art to which the present invention belongs.

The singular forms "a", "an", and "the" include references to the plural unless explicitly stated otherwise in the context.

The term "comprising" means "including" as well as "consisting", e.g., a composition "comprising" X may consist exclusively of X or may include something additional, e.g., X + Y.

The term "epicardioid" is used herein interchangeably with the term "heart tissue model," wherein the epicardioid has been isolated at day 13, preferably at day 15 or later of the culture, and wherein the epicardioid shows the disclosed characteristic structure. Epicardioid is the given name for the herein described mature heart tissue model.

The term "early epicardioid" is used for epicardioids which are isolated from culture at day 1, 2, 3, 4, 5, 6, 7, 8, 9, 10, 11, or 12 of the culture, preferably between day 6 and 10 of the culture, i.e., prior to the day of full epicardioid development at day 13 of the culture. Such early epicardioids are nevertheless also heart tissue models of the present invention.

The term "about" means, in terms of numbers, concentrations, times (minutes, hours, days) and the like, that +/- 10% of the given number, concentration, time (minutes, hours, days) and the like is also comprised. For example, if a certain agent is present in an amount of about 2 µM to about 4 µM in a medium, the agent may be present in an amount of 2 µM (+/- 10%) to 4 µM (+/-10%) in this medium, i.e. in an amount of at least 1,8 µM to at most 4,4 µM.

The expression "densely packed" means a high density of cardiomyocytes (CM), on average about between 4000-8500 CM/mm², preferably about 6500 CM/mm².

The expression "looser packed" means a lower density of cardiomyocytes (CM), on average about between 3000-6200 CM/mm², preferably about 4500 CM/mm².

The term "compartment" means a defined sub-structure in a higher order structure comprising, for instance, characteristic cell types or a certain pattern or layers of cells, which sub-structure differs from other sub-structures within the higher order structure (here within the epicardioid).

The feature of a "significantly higher" proportion of cells of a specific cell type, e.g., Ki67+ cardiomyocytes, for instance, in a compartment or layer, means that cell numbers of this cell type statistically significantly differ from cell numbers of the same cell type in, e.g., another compartment or layer. In embodiments this means that cell numbers of a certain cell type are at least 40%, 50%, 60%, 70%, 80%, 90%, 100%, 150%, 200%, 250%, 300% or 350% higher compared to cell numbers of the same cell type in, e.g., another compartment or layer.

The term "x days of culture", the expression "at day x", or "at day x of differentiation" can be used interchangeably and mean that the epicardioids were isolated and analyzed after x days of culture.

### DETAILED DESCRIPTION OF THE INVENTION

The epicardium is the mesothelial cell sheet covering the heart's outer surface. Long considered a simple barrier between the pericardial cavity and the myocardium, it is now recognized to hold key functions in cardiac development and repair. During embryonic development, a subset of epicardial cells undergoes epithelial-to-mesenchymal transition (EMT) to become epicardial derived cells (EPDCs) that migrate into the myocardium and give rise to the majority of fibroblasts and vascular smooth muscle cells of the heart. Whether EPDCs also differentiate into cardiomyocytes and coronary endothelial cells is still debated, with studies providing conflicting evidence. In addition to these cellular contributions, the epicardium provides signaling factors critical for the development and growth of the myocardium. It also plays a central role in heart regeneration in species capable of rebuilding adult heart muscle upon injury such as the zebrafish, making it a highly promising target for therapy. However, the inaccessibility of human embryonic tissue at the early stages of epicardium development, which begins less than four weeks post-conception, has left significant gaps in our understanding of human epicardial development and function. Many questions on the ontogeny of human proepicardial precursors and the functional heterogeneity of epicardial cells are still unresolved, which limits harnessing their full potential for regenerative medicine.

Pluripotent stem cell-derived cardiac organoids have emerged as powerful *in vitro* models of human development and disease, but none have yet demonstrated the formation of a *bona fide* epicardial compartment. The present invention provides cardiac organoids showing self-organization of highly functional ventricular myocardium and epicardium, which were thus called epicardioids. As evidenced in the disclosed Experiments, time course single-cell genomics in epicardioids revealed principles of human epicardial biology, including lineage heterogeneity and functional crosstalk with other cardiac cell types. In addition, the herein disclosed epicardioids represent an advanced system to model multicellular mechanisms of heart disease.

The invention is described in detail below. All embodiments can be combined with each other, except where otherwise stated.

The inventions disclosed herein do not concern processes for modifying the germline genetic identity of human beings. The inventions disclosed herein do not concern uses of human embryos for industrial, commercial or any other purposes. The inventions disclosed herein do not concern the human body at the various stages of its formation. The pluripotent stem cells used herein for the production of the epicardioids and thus used in the inventive methods are not obtained by means of a process in which human embryos are destroyed.

### Epicardioids

The invention relates to a heart tissue model named epicardioid. Such epicardioid has a three-dimensional, sphere-like shape comprising two different compartments, the inner myocardial compartment forming the "core" of the epicardioid, and the outer epicardial compartment, wrapped around the core, i.e., forming an envelope of the core. Each compartment comprises different cell layers *(see* **Fig.** 1). The outer epicardial compartment comprises from the outside to the inside
- a layer of epicardium comprising KRT18+ mesothelial epicardial cells, and
- a layer of subepicardium comprising Vim+ epicardial-derived cells (EPDCs).

The inner myocardial compartment comprises from the outside to the inside
- a layer of compact-like myocardium comprising densely packed cTNT+ myocardial cells, and
- a layer of trabecular-like myocardium comprising looser packed cTnT+ myocardial cells compared to the compact-like myocardium.

All cells present in the epicardioid have been developed from one initial *in vitro* cell population. This means, that no cells were added to the epicardioid at a later point after the initial cell population has been seeded for epicardioid generation according to the inventive methods. Therefore, a disclosed epicardioid is no artificial composition of different pre-existing cell types put together *in vitro* but resembles naturally developped embryonic heart tissue.

cTnT is the sarcomeric marker cardiac troponin T. Vim is the abbreviation for the mesenchymal marker vimentin. Although Vimentin is an established mesenchymal marker, it can be expressed in the epicardium.

The initial *in vitro* cell population is an *in vitro* pluripotent stem cell population comprised in cell culture medium, wherein the pluripotent stem cell population is preferably an induced pluripotent stem cell population, more preferably a human induced pluripotent stem cell population, or wherein the pluripotent stem cell population is preferably an embryonic stem cell population, more preferably a human embryonic stem cell population. In another embodiment, the pluripotent stem cell population is a human induced pluripotent stem cells reprogrammed from patients with genetic or complex cardiac disorders, including congenital heart diseases. In a preferred embodiment, the epicardioid has been developed from one single population of human induced pluripotent stem cells.

The trabecular-like layer may also be seen as a trabecular-like inner core of the three-dimensional epicardioid.

The cells of the outer epicardial compartment may express, in addition to KRT18, the epicardial transcription factors TBX18.

The cells present in the layer of compacted myocardium preferably comprise a significantly higher proportion of Ki67⁺ proliferating cardiomyocytes than the inner myocardium. Significantly higher in this specific case means that the proportion of Ki67+ cells in the outer compact layer is between about 100%-350%, more specifically between 129%-309% higher than the proportion of Ki67+ cells in the inner trabecular-like layer.

The inner myocardial compartment may further comprise in both layers non-cardiomyocyte cells comprising cardiac fibroblasts, vascular smooth muscle cells and endothelial cells. The endothelial cells may be CD31⁺.

The two compartments of the epicardioid preferably match the multilayered structure of the ventricular epicardium and myocardium of early human embryos.

### Use of epicardioids

The epicardioids disclosed herein may be used as a heart tissue model. Therefore, the present invention also relates to the use of the disclosed epicardioids as a heart tissue model.

Epicardioids are formed through processes that mimic the development of the left ventricle during embryonic development, including the specification of TBX5+ cells corresponding to first heart field (FHF) progenitors. To our knowledge, they are also the first human system showing the existence of juxta-cardiac field (JCF) cells recently discovered in the mouse as an early progenitor of both myocardium and epicardium (Tyser et al., (2021), "Characterization of a common progenitor pool of the epicardium and myocardium", Science, 80:eabb2986; Zhang et al., (2021) "Unveiling Complexity and Multipotentiality of Early Heart Fields", Circ. Res. 129:474-487). The presence of the epicardium in epicardioids promotes morphological, molecular, and functional patterning of the myocardium that was previously not achieved in cardiac *in vitro* models. Specifically, as evidenced herein in the Examples, the epicardium stimulates cardiomyocyte proliferation via cell-cell interactions that have been described in human embryos.

Epicardioids therefore offer a unique platform to investigate mechanisms underlying epicardial lineage formation and the functional crosstalk between the epicardium and myocardium (e.g. via transcriptomic and proteomic analyses of epicardioids at relevant time points, lineage tracing studies with reporter hPSC lines, generation of epicardioids from genetically engineered hPSC lines to modify specific processes).

Notably, insights from these studies could lead to novel strategies to re-activate the epicardial capacity to stimulate cardiomyocyte proliferation, which is lost in adults, to replace cardiomyocytes lost after injury (e.g. myocardial infarction).

Therefore, in one aspect, the herein disclosed epicardioids are used for studying heart diseases. The inventive epicardioid may be used to recapitulate specific normal or abnormal conditions of the heart, such as diseases, illnesses or injuries, as well as a recovery from such conditions.

In one embodiment, the heart disease is a left ventricular heart disease.

In another preferred embodiment, the herein disclosed epicardioids are used for preclinical testing of drugs for the treatment of heart diseases. Such heart diseases may be selected from hypertrophic, fibrotic, infective, or arrhythmic or other heart diseases. In even another embodiment, the epicardioids are used for preclinical testing of drug-induced arrhythmias.

Moreover, also encompassed by the invention is the use of epicardioids generated from human induced pluripotent stem cells reprogrammed from patients with genetic or complex cardiac disorders, comprising congenital heart diseases, for studying the mechanisms of disease and for testing drugs to rescue disease phenotypes. The epicardioids can thus be used for testing genetic alterations, for instance by observing the effects of mutated (e.g., disease-related mutations), suppressed or over-expressed genes during heart development wherein the testing comprises the production of a heart tissue model (epicardioid) according to the disclosed method, wherein the cells have a suppressed candidate gene or overexpress a candidate gene or have a mutation in a gene and comparison of the development of the heart tissue model with the development of a heart tissue model not having such genetic alteration. Mutation, overexpression or suppression of expression can be done with any known method in the art, such as suppression by gene knock out, siRNA inhibition or CRISPR/Cas-based inactivation. Overexpression can be done by introduction of a transgene or applying a gene activator that leads to upregulation of expression of a gene.

In another embodiment, the herein disclosed epicardioids are used for investigating disease states and their treatment by application of pharmacological stimuli to epicardioids. In a preferred embodiment, the herein disclosed epicardioids are used to study left ventricular hypertrophy, wherein the epicardioids are treated with endothelin-1.

Candidate drugs or substances identified in epicardioids would need to be tested in an animal model of cardiac disease or injury (e.g. myocardial infarction). This may be achieved by delivery of recombinant proteins or small molecules, or by gene activation or inhibition via CRISPR/Cas9 technology. Different methods of delivery could be envisioned, including intrapericardial, intramyocardial, and endovascular injection and engraftment of a patch (e.g. collagen, fibrin) loaded with appropriate factors at the site of injury.

### Methods of producing heart tissue models

The inventive tissue models or epicardioids are the results of the inventive methods disclosed herein, or can be used in the inventive methods and for the inventive uses.

All cell comparisons herein are under standard conditions for cell cultures for maintaining cells expressing relevant distinctive markers or signature marker expression, in particular so that the markers or marker expression can be determined as a cell-intrinsic property. Such conditions usually comprise a medium consisting of all required nutrients for maintenance under ambient pressure and at the cells' physiological temperature.

The inventive heart tissue model is artificial and grown in culture using natural principles of development. By following the inventive method steps the heart tissue model in form of an epicardioid is generated by self-organization driven by growth factor signaling.

The method of producing a three dimensional heart tissue model comprises the following consecutive steps:
First pluripotent stem cells are provided and seeded in an initial culture medium on day -1 of the culture.

The pluripotent stem cells (PSCs) are preferably from a mammal. Preferably the PCSs are human PSCs or non-human primate PSCs, or PCSs from a rodent, mouse, hamster, pig, cat, dog, horse, or cow. The PSCs may be from a cell line or a cell culture. Preferably, the PSCs are induced pluripotent stem cells (iPSCs). iPSCs stem from differentiated cells that were again turned pluripotent, such as by treatment with the Yamanaka factors. Such iPSCs are preferably human iPSCs (hiPSCs). Usage of iPSCs allows for the investigation of cardiac development of specific individuals, which may or may not have genetic aberrations that may affect heart development. The iPSCs may therefore be developed from cells of healthy or diseased subjects, preferably humans, wherein a diseases subject for instance suffers from a heart disease, in particular a genetic heart disease. Alternatively, the pluripotent stem cells are embryonic stem cells (ESCs), preferably human embryonic stem cells (hESCs) of a hESC cell line. Of note, when using ESCs, no embryos have been destroyed.

The PSCs are grown in a medium before they are provided for the steps of the inventive method. This initial culture medium is preferably Essential 8 (E8) medium. Preferably, the cells are cultured on Geltrex-coated plates in E8 medium.

At day -1 of the culture, the PSCs are detached from the culture plate, washed and counted. This PSC cell culture is then provided for the first step of the inventive method. The cells are seeded in an initial culture medium immediately after cell counting, i.e., on day -1. The initial culture medium used for cell seeding is preferably an E8 medium comprising thiazovivin. Preferably, the E8 medium comprises about 1 to about 5 µM thiazovivin, more preferably about 2 µM thiazovivin, most preferably 2 µM thiazovivin.

In an embodiment, the PSCs are seeded in low attachment culture vessels. Such vessels are preferably U-shaped or V-shaped. In a preferred embodiments the PSCs are seeded in wells of a U-shaped 96-well plate. Low attachment culture vessels prevent the PSCs from attaching to the surface of the bottom of the vessels and thus supports the cells to grow in spheres, i.e. it supports a 3D culture. Low attachment surfaces are preferably hydrophilic, neutrally charged or non-ionic. They may have a hydrogel layer or coating that repels cell attachment, forcing cells into a suspended state. In preferred embodiments, the vessels are coated with a low attachment agent or low cell adherence agent. Such agent is for instance poly-HEMA. In a preferred embodiment, the PSCs are seeded in U-shaped wells of a poly-HEMA-coated U-shaped 96-well plate.

A preferred starting cell number seeded to generate the heart tissue models of the present invention is between about 30,000 and about 40,000 cells, i.e., about 30,000, about 30,500, about 31,000, about 31,500, about 32,000, about 32,500, about 33,000, about 33,500, about 34,000, about 34,500, about 35,000, about 35,500, about 36,000, about 36,500, about 37,000, about 37,500, about 38,000, about 38,500, about 39,000, about 39,500, or about 40,000 cells. Too low numbers may lead to structures that do not start spontaneously beating, indicating failed cardiac differentiation. Of note, epicardioids of the present invention start spontaneously beating around day 8 of culture. On the other hand, structures generated from too high numbers may not acquire the correct morphology in the early stages of epicardioid formation.

In a preferred embodiment, 30.000-40.000 cells are seeded in 150 µl of culture medium in a U-shaped well of a poly-HEMA coated 96-well plate.

Day -1 means about 20-28 hours, preferably about 24 hours, before starting the cell differentiation steps.

On day 0 of the culture, the culture medium is replaced with a first basal differentiation medium.

Day 0 means 20-28 hours after seeding the PSCs. The culture is preferably replaced with the first basal differentiation medium about 24 hours after seeding, more preferably 24 hours after seeding.

The first basal differentiation medium preferably comprises bone morphogenetic protein 4 (BMP4), Activin A, fibroblast growth factor beta (bFGF, also called FGF-β or FGF2), a PI3-Kinase inhibitor, preferably LY-29004, and a Wnt activator, preferably CHIR-99021.

The first basal differentiation medium is preferably based on a basic basal differentiation medium, which is further supplemented with the components of the first basal differentiation medium.

The basic basal differentiation medium preferably comprises a standard medium suitable for PSC differentiation cell culture, such as DMEM. Preferably DMEM is mixed about 1:1 with IMDM. Preferably the basic basal differentiation medium further comprises BSA, chemically defined lipid concentrate, transferrin, α-monothioglycerol. Preferably the basic basal differentiation medium further comprises about 1% chemically defined lipid concentrate, about 0,05% transferrin, about 0,04% α-monothioglycerol.

The first basal culture medium used for the first medium replacement step of the method, may be the basic basal differentiation medium described above, which is further supplemented with preferably BMP4, Activin A, bFGF, LY-29004, CHIR-99021, more preferably with about 10 ng/mL BMP4, about 50 ng/mL Activin A, about 30 ng/mL bFGF, about 5 µM LY-29004, and about 1,5 µMCHIR-99021.

On day 2 of the culture, the culture medium is replaced with a second basal differentiation medium comprising retinoic acid. The retinoic acid is preferably present at a concentration of 0,3-0,75 µM, preferably of about 0,5 µM, preferably of 0,5 µM.

Day 2 of the culture means about 40 to 50 hours after the seeding of the cells, preferably, day 2 means about 40-42 hours after seeding.

The second basal differentiation medium preferably further comprises insulin, BMP4, bFGF, and a Wnt antagonist, preferably IWP2, more preferably about 10 ng/mL insulin, about 10 ng/mLBMP4, about 8 ng/mL bFGF and about 5 µM IWP2.

Preferably, the second basal differentiation medium is the basic basal differentiation medium further supplemented with about 0,5 µM retinoic acid, about 10 µg/mL insulin, about 10 ng/mLBMP4, about 8 ng/mL bFGF and about 5 µM IWP2.

This culture medium is replaced with fresh second basal differentiation medium each 24 hours until day 6 of the culture.

Next, on day 6 of the culture, the medium is replaced with a third basal differentiation medium.

Day 6 of the culture means about 134 to 144 hours after the seeding of the cells, preferably, day 6 means about 136-138 hours after seeding.

The third basal differentiation medium preferably comprises insulin, BMP4, and bFGF, more preferably about 10 µg/mL insulin, about 10 ng/mLBMP4, and about 8 ng/mL bFGF.

Preferably, the third basal differentiation medium is the basic basal differentiation medium further supplemented with about 10 µg/mL insulin, about 10 ng/mLBMP4, and about 8 ng/mL bFGF.

This culture medium is replaced with fresh third basal differentiation medium on day 7 of the culture.

On day 8 of the culture, the spheroids are embedded into a gel comprising collagen type I. For this, a pipet tip may be used to transfer the spheres into molds in the gel. The molds are subsequently filled with collagen-I solution which was then allowed to solidify. This gel sheets are then transferred to maintenance medium, i.e., they are surrounded by maintenance medium.

Day 8 of the culture means about 184-194 hours after seeding of the cells, preferably about 184-188 hours after seeding of the cells.

The maintenance medium preferably comprises insulin and VEGF, more preferably, about 10 µg/mL insulin and about 100 ng/mL VEGF.

Preferably, the maintenance medium is the basic basal differentiation medium further supplemented with about 10 µg/mL insulin and 100 ng/mL VEGF.

The maintenance medium is replaced with fresh maintenance medium every 2-3 days after day 8 of the culture.

Optionally, the gel sheets floating in maintenance medium are placed on a rocking shaker on day 10 of the culture.

Day 10 of the culture means about 230-240 hours after seeding the cells, preferably about 230-236 hours after the seeding of the cells.

As a last step, the spheroids, which are now called epicardioids can be isolated from the gel sheets for further use. Isolation of epicardioids can take place starting on day 13 of the culture.

If the maintenance medium is replaced all 2-3 days of the culture after day 8, the epicardioids can be cultured for longer time, for instance until day 100 of the culture. Preferably, epicardioids are isolated between days 13 and 100 of the culture, more preferably, between days 15 and 40 of the culture.

During embryonic cardiac development, the epicardium secretes factors that stimulate cardiomyocyte proliferation, thereby promoting the formation of a compact layer that is essential for proper cardiac function. By following the disclosed method, this is recapitulated in the end product of the disclosed method, which is a heart tissue model named epicardioid, which consistently forms an inner myocardial core (the trabecular-like layer) an outer region of higher cardiomyocyte density of approximately 50 µm width (the compact-like layer).

The molecular processes taking place during initial epicardioid formation when following the herein disclosed method have been analyzed based on single-cell RNA sequencing at days 2, 3, 4, 5, 7, 10 and 15 (*see* **Fig. 2A****,B).** This revealed early induction of cardiac mesoderm (marked by MESP1) followed by cardiac progenitor cells predominantly corresponding to the first heart field (marked by *TBX5)* **(****Fig. 2C****).** Cardiomyocytes started expressing the ventricular marker *MYL3* from day 7; by day 15 most cardiomyocytes matched to a human ventricular signature (co-expression of *MYH7, MYL2, S100A4, VCAN, MASP1)* **(****Fig. 2C****,D).** On day 15, there was also a cluster matching a human epicardial signature (co-expression of *SULF1, TM4SF1, CCDC80, S100A10, CFI*) (**Fig. 2D**).

In earlier epicardioids, a pro-epicardial progenitor population could be identified which corresponds to the juxta-cardiac field reported in mice as a common progenitor or the myocardium and epicardium (marked by *HAND1, MAB21L2, HOXB6, HOXB5, BNC2)* - this is the first time that this population has been described in a human system (**Fig. 2D**). Ongoing trajectory analyses suggest that this population gives rise to epicardial cells that can differentiate into fibroblasts and smooth muscle cells, as well as a small cardiomyocyte population (data not shown).

Based on these observations the present invention also relates to a method for screening or testing a candidate compound on its effects on heart development and/or functionality.

This screening method is based on the inventive culture method disclosed herein for generating a heart tissue model by performing the disclosed method steps while treating the cells with the candidate compound and comparing development of the heart tissue model with development and/or functionality of a heart tissue model that was not treated with the candidate compound. For a successful analysis all culture steps should be the same except the treatment with a candidate compound or drug.

The method may also comprise a step of causing an injury to the heart tissue model and optionally further a recovery from such injury. Agents or compounds may be tested to cause tissue injury or a diseased state of the tissue. Alternatively, such compounds may be tested for their effect during an injury or their effect on the healing process.

For getting insights into effects of the certain compound in early cell differentiation, heart tissue models can be isolated before having completed all method steps. Therefore, the spheres present on day 1, 2, 3, 4, 5, 6, 7, 8, 9, 10, 11, or 12 of the culture, preferably present at any day prior to day 10 of the culture, can be isolated for further analysis. These isolated spheres are herein also referred to as heart tissue models, or "early epicardioids". The term "epicardioid", however, is only used for heart tissue models which are isolated only after the completion of all method steps of the method disclosed herein. So there are several possible end-products called "heart tissue model" or "early epicardioid" but only one end-product called "epicardioid", wherein the epicardioid is isolated at day 10 or later, preferably at day 15 or later.

### Heart tissue model obtained by the inventive method

The present invention also relates to a heart tissue model obtained by the herein disclosed inventive method(s). The present invention therefor provides a heart tissue model which has been prepared according to the disclosed method.

In preferred embodiments, the heart tissue model is an epicardioid, as described herein, comprising its characteristic structure in terms of cellular compartments.

In another embodiment, the heart tissue model is an early epicardioid, which has been isolated from culture before day 10, for example, at day 1, 2, 3, 4, 5, 6, 7, 8 or 9 of the culture, wherein the culture protocol does not differ from the method disclosed herein. Isolating early epicardioids may be used for analysis of cell development at different stages cell differentiation.

### EXAMPLES

The catalog number of commercially available reagents and consumables is indicated; it belongs to the routine work of the skilled person that all reagents mentioned herein could likely be replaced with equivalent products from other companies without affecting the outcome. All steps are performed using sterile technique.

### hPSC culture

Different cell lines have been used for the Examples disclosed herein. The following hiPSC lines were used: hPSCreg MRI0003-A (hiPSC1), MRI001-A (hiPSC2), and MRI003-A-6 (AAVS1-CAG-VSFP; hiPSC3). For ESCs, the hESC cell line HES-3 (hPSCreg ESIBIe003) was used.

Prior to epicardioid differentiation, hPSCs are maintained on Geltrex-coated (Gibco; A14133-02) 35 mm tissue culture plates (Falcon; 353001) in Essential 8 medium (E8; Gibco; A1517001). Cells are passaged at a ratio of 1:14-1:18 every 4 days with 0.5 mM EDTA in PBS without Ca²⁺ or Mg²⁺ (PBS^{-/-}; Gibco; 10010023). The ROCK inhibitor Thiazovivin (TV; Sigma-Aldrich; SML1045) is added at a concentration of 2 µM for 24 h after passaging to promote survival.

### Example 1: generation of epicardioids

### Preparation of basal medium

The basal differentiation medium is prepared by mixing 247.36 mL DMEM/F-12 with GlutaMAX (Gibco; 31331028), 237.36 mL IMDM (Gibco; 21980032), 5 mL chemically defined lipid concentrate (Gibco; 11905031), 10 mL IMDM containing 10% BSA (GE Healthcare; K41-001), 250 µL transferrin (Roche; 10652202001), and 20 µL α-monothioglycerol (Sigma-Aldrich; M6145). The basal medium can be stored for up to 2 months at 4°C. Complete differentiation media are prepared maximum 2 days in advance and stored at 4°C. Media containing retinoic acid are protected from light. Poly-HEMA-coated plates are prepared by adding 50 µL polyHEMA solution consisting of 5% poly-HEMA (Sigma-Aldrich; P3932) in 95% ethanol (Merck; 1009831000) per well of a U-shaped 96-well plate (Falcon; 351177) and letting them air dry overnight.

### Epicardioid differentiation and maintenance

On day -1, hPSCs having reached ^{~}80% confluency are washed with 1 mL 0.5 mM EDTA in PBS^{-/-}and dissociated for 6-8 min with 1 mL of the same solution at room temperature (RT) (until cells can easily be detached from the plate). After removing the solution, 1 mL E8 medium containing 2 µM TV is added to the plate to detach the cells from the plate by gently pipetting up and down 10-15 times. The cell suspension is transferred to a 50 mL tube (Greiner Cellstar; 227261) and diluted with 1 mL E8 + TV. After counting cells with a Neubauer chamber, 30,000-40,000 cells are then seeded into poly-HEMA-coated U-shaped 96-well plates in a total volume of 150 µL Essential 8 medium with 2 µM Thiazovivin. To ensure that a single spheroid will be formed per well, the plate is centrifuged for 1 min at 300 g at RT before placing in the incubator under standard conditions that are maintained during the entire culture (37°C, 5% CO₂).

On day 0 (roughly 24 h after seeding cells on day -1), the entire medium is replaced with 150 µL basal medium supplemented with 10 ng/mL BMP4 (R&D; 314-BP), 50 ng/mL Activin A (Sigma-Aldrich; SRP3003), 30 ng/mL FGF2 (R&D; 233-FB-025/CF), 5 µM LY-29004 (Tocris; 1130), and 1.5 µM CHIR-99021 (Axon Medchem; 1386).

Between 40 and 42 h after day 0 (referred to as day 2), the medium is replaced with 150 µL basal medium supplemented with 10 µg/mL insulin (Sigma-Aldrich; 19278), 10 ng/mL BMP4, 8 ng/mL FGF2, 5 µM IWP2 (Tocris; 3533), and 0.5 µM retinoic acid (Sigma-Aldrich; R2625), which is refreshed every 24 h on days 2 to 5.

On day 6, the medium is replaced with 150 µL basal medium supplemented with 10 µg/mL insulin, 10 ng/mL BMP4, and 8 ng/mL FGF2, which is refreshed 24 h later on day 7.

On day 8, the spheroids are embedded into a gel containing collagen type I. For this, the edges of 15 mm × 15 mm × 5 mm molds (Tissue-Tek: 4566) are trimmed with scissors to fit into the wells of a 6-well culture plate (Nunc; 140675) and the molds are sterilized with 70% ethanol. A cut 1,000 µL pipet tip is then used to transfer the spheroids into the molds placed in the 6-well plate. Any transferred medium is then removed and the molds are filled with 400 µL collagen I solution consisting of 2.17 mg/mL collagen I (Corning; 354249), 20% distilled water (Gibco; 15230162), 5% 10× DPBS (Gibco; 14080055), and 8.3 mM NaOH in EB20 medium consisting of DMEM/F-12 with 20% fetal bovine serum (Sigma-Aldrich; F7524), 1% non-essential amino acids (Gibco; 11140050), 1% Penicillin-Streptomycin-Glutamine (Gibco; 10378016), and 0.1 mM β-mercaptoethanol (Sigma-Aldrich; M7522) prepared on ice. After briefly distancing the spheroids from each other using a pipette tip and closing the lid of the 6-well plate, they are placed at 37°C for 30 min until the collagen solidifies. The gel sheets obtained are then carefully removed from the molds by adding 1 mL maintenance medium consisting of basal medium supplemented with 10 µg/mL insulin, 0.5% Penicillin-Streptomycin (Gibco; 15140-122) and 100 ng/mL VEGF (R&D; 293-VE-010) per well and slowly pipetting 1 mL medium under the sheets to make them slide into the well.

On day 10, the medium is refreshed and the plates are placed on a rocking shaker (Assistant, Germany) at 40 rpm to improve oxygen and nutrient diffusion.

For long-term culture, the maintenance medium is refreshed every 2-3 days.

This protocol has been successfully applied to several iPSC and ESC lines, comprising hPSCreg MR1003-A (hiPSC1), MR1001-A (hiPSC2), MR1003-A-6 (AAVS1-CAG-VSFP; hiPSC3), and HES-3 (hPSCreg ESIBISe003; hESC) (see, **Fig. 3**).

### Example 2: Harvesting epicardioids

Epicardioids are harvested by gently pushing them out of the collagen sheet into the culture medium, for example with a pipette tip. They can then be transferred to other containers using a pipette, e.g. for fixation or dissociation (see Materials and Methods).

### Example 3: Epicardioids recapitulate the molecular, morphological, and functional patterning of the ventricular wall

To further investigate the composition of epicardioids, we performed whole-transcriptome analysis by single-cell RNA-sequencing (scRNA-seq) at days 15 and 30 (**Fig. 4E**). Unsupervised cluster analysis revealed that the most abundant cells were ventricular cardiomyocytes, which showed a higher ratio of adult to fetal cardiac troponin I isoforms (TNNI3/TNNI1) as well as increased expression of Ca2+ handling genes (ATP2A2, SLC8A1, PLN) at day 30 compared to day 15, indicating progressive maturation (**Fig. 4F****,G;** **Fig. 5A**). Interestingly, there was a small cardiomyocyte cluster (cluster 8) showing high SHOX2 and HCN4 expression, suggesting pacemaker identity (**Fig. 5B**). By comparison with recently available sequencing datasets from human fetal epicardium, we identified three clusters sharing epicardial markers (**Fig. 6A**). The first (cluster 11) comprised mesothelial epicardial cells, which expressed epithelial genes and showed progressive upregulation of specific human epicardial markers (TNNT1, C3, CALB2) (**Fig. 4H****;** **Fig. 6B**). The second largest cluster (cluster 5) contained EPDCs expressing markers of EMT and mesenchymal epicardium (**Fig. 4H****;** **Fig. 6C**). It also comprised epicardium-derived cardiac fibroblasts and vascular smooth muscle cells (**Fig. 4H****;** **Fig. 6D****,E**). This cluster showed no notable differences between the two time points, suggesting a continuous capacity of the mesothelial epicardium to undergo EMT and further differentiation (**Fig. 4H****;** **Fig. 6C-E**). The third epicardial cluster (cluster 10) resulted from the segregation of highly proliferative cells of both mesothelial and mesenchymal identity (**Fig. 6F**). Finally, a cluster of endothelial cells (cluster 12) was identified (**Fig. 7**).

Inferring cell-cell communications from the scRNA-seq dataset revealed ample interaction between epicardial cells and other cell types in epicardioids (**Fig. 8A**). The volume of signaling from mesothelial epicardium to cardiomyocytes noticeably declined over time, however, hinting to a progression towards the quiescent state characteristic of adult epicardium (**Fig. 8A**). Epicardial signals known to stimulate cardiomyocyte proliferation in the mouse, such as the secretion of IGF2 and fibronectin binding to integrin β1, as well as the CXCL12-CXCR4 axis promoting coronary angiogenesis in zebrafish were detected (**Fig. 8B**). Epicardioids also recapitulated ligand-receptor interactions described between epicardial cells and cardiomyocytes (NRP2_VEGFA) or endothelial cells (MIF_TNFRSF10D) in human embryos (**Fig. 8B**). Although most interactions were common between mesothelial epicardium (cluster 11) and epicardium-derived cells (cluster 5), signals specifically described in adult mesothelial epicardium, such as binding of vitronectin (VTN) to αvβ1 integrin, were exclusive to the mesothelial cluster **(****Fig. 8B****,** **Fig. 9****).**

*In vivo,* epicardial stimulation of cardiomyocyte proliferation contributes to the formation of a subepicardial compact myocardium that is molecularly and functionally distinct from the trabecular myocardium facing the ventricular lumen. Opposing gradients in the expression levels of genes enriched in human compact (FTH1, FTL, COL3A1) and trabecular (COL2A1, TTN, MALAT1) myocardium suggested an equivalent patterning in epicardioids, as we could distinguish between clusters with compact, trabecular, and intermediate transcriptional profiles (**Fig. 8C**). This was further corroborated by the presence of an approximately 50 µm-wide zone of higher cardiomyocyte density underneath the epicardial layer from day 15, which mimicked the morphology of human fetal tissue (**Fig. 3A-C**). Of note, this was not observed in spheroids differentiated without RA (noRA) lacking the epicardial layer (**Fig. 10B****,C**). Consistent with the molecular mechanisms described above, the compact layer of epicardioids had a significantly higher proportion of Ki67+ proliferating cardiomyocytes than the inner myocardium (**Fig. 10D****,E**).

It was next evaluated if this morphological patterning was associated with regional differences in cardiomyocyte function. In the heart, cardiomyocytes closest to the epicardium generate shorter action potentials (AP) than those in the middle of the ventricular wall, an evolutionary conserved feature increasing the efficiency of ventricular contraction, which is known as the transmural voltage gradient. To assess AP dynamics across the myocardial compartment in epicardioids, we generated them from hPSCs constitutively expressing a FRETbased voltage sensor knocked into the AAVS1 safe harbor locus (AAVS1-CAG-VSFP; **Fig. 10F**). Optical measurements of AP duration across 250 µm-thick slices prepared by vibratome sectioning revealed significantly shorter durations to 50% and 90% repolarization in cardiomyocytes of the subepicardial layer compared to the inner myocardium at day 35 (**Fig. 10G****,H**). As excitation-contraction coupling is dependent on the intracellular dynamics of calcium, calcium imaging was performed with the fluorescent indicator Fluo-4 (**Fig. 10I**)**.** This showed a corresponding pattern of shorter durations to 50% and 90% peak decay in the subepicardial layer compared to the inner layer (**Fig. 10J**). Neither of these functional gradients were observed in age-matched noRA spheroids, confirming that they are not intrinsic properties of cardiac spheres (**Fig. 10G****,H,J**).

### Example 4: Epicardioids formed by early segregation of first heart field and juxta-cardiac field progenitors

Animal models have shown that the epicardium is formed by cells of the proepicardium, a transient structure located at the venous pole of the looping-stage heart, but the ontogeny of proepicardial precursors in humans is still unclear. To dissect the developmental processes taking place in epicardioids, we performed scRNA-seq in parallel with chromatin accessibility profiling via scATAC-seq at days 2, 3, 4, 5, 7, 10, and 15.

Combined analysis of the 31 cell clusters obtained by scRNA-seq revealed early induction of cardiac mesoderm followed by the emergence of cells expressing markers of first heart field progenitors (FHF; TBX5, SFRP5), which mainly contribute to the left ventricle in the mouse (**Fig. 11A**, **12A-C**). Of note, cells expressing markers specific of anterior second heart field precursors (SHF; TBX1, FGF8) - which generate the right ventricle and the outflow tract - were absent (**Fig. 12D**). Differentiated ventricular cardiomyocytes were detected from day 7 and showed progressive upregulation of the mature ventricular marker MYL2 **(****Fig. 11A****;** **Fig. 12A****,B).** In parallel to cardiac development, we observed a small contribution of endodermal cells that was largely lost by day 15 **(****Fig. 11A****;** **Fig. 12A****,B).** Epicardial cells and their derivatives first emerged on day 10 and expanded on day 15 (cluster 23; **Fig. 11A****;** **Fig. 12A****,B).** Interestingly, they were preceded by cells closely matching the transcriptional signature of the juxta-cardiac field (JCF; HAND1, HOXB6, MAB21L2), a common progenitor of myocardium and epicardium recently described as a subset of the FHF in the mouse and not yet identified in humans (clusters 21 and 27; **Fig. 11A****,B,** **Fig. 12A****,B).** Computational reconstruction of the developmental trajectories of cells from day 2 to day 15 suggested that the epicardial lineage as well as a proportion of cardiomyocytes were descendants of these cells, with a small population of cardiomyocytes appearing to derive from epicardial cells themselves (**Fig. 11C****,** **Fig. 13**). It also suggested a distinction between bipotent JCF (for epicardial and myocardial lineages) and unipotent JCF restricted to the myocytic fate (**Fig.** 11C). Both populations were downstream of the same putative "pre-JCF" precursors (cluster 11), which appeared on days 4 and 5 at the same time as classical FHF cells (**Fig. 11A****,C,** **Fig. 12A**). Remarkably, pre-JCF cells expressed the multipotent cardiovascular progenitor marker ISL1 (25) but not the myocytic marker NKX2.5 (**Fig. 14A**). This allowed us to follow their physical segregation from ISL1+/NKX2.5+ FHF progenitors, which was clearly visible by day 5, with preJCF cells already concentrated at the outer layer (**Fig. 14B**). High expression of ISL1 was maintained in the JCF at days 7 and 10, followed by a decrease in the epicardial cluster at day 15 (**Fig. 15A**). This reflected a maintenance of ISL1 solely in the mesothelial epicardium and a loss of expression in subjacent EPDCs (**Fig. 11D**). As ISL1 expression was not previously reported in the epicardium (human or otherwise), ISL1 immunostaining was performed in human fetal heart tissue to verify this finding. This revealed that epicardial cells were indeed positive for ISL1 at 5 weeks post conception, but expression was completely lost by 6 weeks (**Fig. 11D**). We also observed loss of ISL1 in epicardial cells of 30-day-old epicardioids, suggesting equivalent expression dynamics (**Fig. 15B**).

To gain deeper insight into the specification of JCF progenitors in epicardioids and identify putative regulatory programs that could underlie divergence of bipotent and unipotent pools, chromatin accessibility profiles were analyzed via scATAC-seq at days 3, 4, and 5. Combined cluster analysis revealed four main cell types showing dynamic progression over time: cardiac mesoderm (clusters 0 and 12), endoderm (clusters 4, 5, and 10), cells with myocytic commitment (clusters 1, 3, and 11), and cells with (pre-)JCF commitment (cluster 2, 8, 9) **(****Fig. 11E****,** **Fig. 17A****).** The latter were identified by screening for concomitant chromatin accessibility of 54 genes defining pre-JCF and early JCF cells in our scRNA-seq dataset (**Fig. 11F**). Differential enrichment analysis of accessible transcription factor (TF) motifs among clusters revealed a separation within the (pre-)JCF population, with clusters 2 and 8 sharing common motif patterns with cluster 12, while cluster 9 showed similarity to the myocytic cluster 3, suggesting related developmental trajectories (**Fig. 11G****,** **Fig. 17B**). We identified TF motifs showing differential accessibility between clusters 2/8 and 9 and investigated the activity of potential downstream targets of these TFs by inferring gene regulatory networks from the scRNA-seq data using SCENIC. Cluster 9 showed increased accessibility for genes with motifs bound by cardiomyocyte-related TFs such as MEIS1, which displayed regulon activity in both FHF clusters and a subset of the pre-JCF (**Fig. 11G****,H,** **Fig. 18A**). By contrast, clusters 2 and 8 presented increased accessibility for genes with motifs bound by TFs showing regulon activity exclusively in the pre-JCF, such as TFAP2A (**Fig. 11G****,H,** **Fig. 18B**). Analysis of peaks at cell type specific loci further indicated that cluster 9 had open chromatin at the transcription start sites of myocytic (NKX2.5) but not epithelial (KRT7) genes, while clusters 2 and 8 had open chromatin for both, denoting potential for both cardiomyocyte and epicardial specification (**Fig. 11I**). Taken together, these findings support the existence of a unipotent myocytic JCF population closely related to the FHF and a bipotent JCF population, which are governed by distinct gene regulatory programs.

### Example 5: Chromatin accessibility patterns uncover dynamic lineage potential in epicardial cells

Beyond their embryonic origin, there are still many open questions concerning the molecular and functional heterogeneity of epicardial cells, which have important implications on potential epicardial re-activation as a therapeutic target. It is debated whether the lineage fate of EPDCs is pre-determined at the (mesothelial) epicardial stage or if specification occurs after EMT. Moreover, it is not known whether there are subsets of epicardial cells that are permissive for opposing cell fates prior to their lineage commitment. Inferring the trajectory of the epicardial clusters in our scRNA-seq dataset at days 7, 10, and 15 revealed diverging fates for mesothelial epicardial cells (KRT19, ISL1) downstream of JCF progenitors (**Fig. 19A****,B**). There was a clear segregation between epicardial cells maintaining their mesothelial identity at day 15 and those giving rise to EPDCs eventually differentiating into fibroblasts and smooth muscle cells (**Fig.19B****,C**). Surprisingly, there was also an entirely distinct branch showing myocytic commitment, which supported the existence of epicardium-derived cardiomyocytes in our system (**Fig. 19C**).

We next investigated whether the mechanisms of fate determination vary between different epicardium-derived cell types by studying chromatin accessibility patterns associated with epicardial differentiation in the scATAC-seq data of days 7, 10, and 15 (**Fig. 20A**). Subclustering of cells of the JCF and epicardial lineage, identified via the corresponding scRNAseq signature, revealed four cell populations (**Fig. 19D, Fig. 20B**). Analysis of differential gene activity allowed us to distinguish between epicardial cells associated with EMT (cluster 0), EPDCs and their derivatives (cluster 1), muscle-fated epicardial cells and EPDCs (cluster 2), and mesothelial JCF/epicardium (cluster 3) (**Fig. 20C**). Aligning cells in pseudotime based on inferred gene activity along the differentiation trajectory revealed that JCF cells on day 7 as well as epicardial cells with mesothelial identity from day 10 and 15 located at the beginning of the trajectory, with no progression in pseudotime, suggesting that they may represent a maintained progenitor pool. This was further supported by high activity of ISL1 and other genes involved in self-renewal (YAP1) **(Fig. 19E,F,** **Fig. 21****).** A vast majority also presented high accessibility for all three lineage-specific markers, namely TNNT2 (cardiomyocyte), MYH11 (smooth muscle), and DDR2 (fibroblast), indicating that they are indeed a multipotent population (**Fig. 19E-H,** **Fig. 21**). A similar mesothelial subset, which showed lower accessibility for fibroblast genes, appeared transiently at day 10 (**Fig. 19E-H,** **Fig. 21**). Cells further along the trajectory showed progressive loss of CDH1 activity, indicating that they undergo EMT. This was associated with dynamic changes in the activity of lineage-specific markers resulting in EPDCs maintaining activity only for one or two lineage markers towards the end of the trajectory, reflecting a more restricted differentiation potential (**Fig. 19E-H,** **Fig. 21**). Interestingly, a reduction in TNNT2 activity could also be observed from day 7 to day 15 in the mesothelial epicardial cells located at the beginning of the trajectory, which was paralleled by an increase of DDR2 accessibility (**Fig. 19G,H**). This points to a progressive loss of cardiomyocyte potential of the mesothelial cells in favor of the fibroblast fate in this population over time, which is in line with the lack of cardiomyocyte potential of adult human epicardium. Overall, our data does not support the existence of discrete subsets of embryonic epicardial cells restricted to a single lineage before EMT, but rather advocates a model of dynamic fate specification over time.

### Example 6: Epicardioids model multicellular processes of left ventricular hypertrophy and remodelling

Cardiac stressors such as high blood pressure or valvular heart disease can lead to left ventricular hypertrophy (LVH) and fibrosis, a maladaptive remodeling of the myocardium that increases patients' risk for heart failure and life-threatening arrhythmia. Current 2D *in vitro* models largely recapitulate the myocytic features of LVH, but fail to account for the pivotal role of fibrosis in the progression towards heart failure. Hypothesizing that the 3D multilineage architecture of the herein disclosed epicardioids could resolve this gap, 1-month-old epicardioids were treated with endothelin-1 (ET1), a potent vasoconstrictor known to induce hypertrophy *in vivo* and *in vitro.* ET1 triggered a dose-dependent upregulation of myocytic hypertrophy markers (NPPA, NPPB, ACTA1, MYH7/MYH6) and an increase in cardiomyocyte size (**Fig. 22A,B**). The concomitant upregulation of ECM genes (COL1A2, COL3A1, FN1, POSTN) suggested that epicardioids do have the capacity to mount a fibrotic response (**Fig. 22C**). This was corroborated by abundant ECM deposition in the subepicardial space and the emergence of α-SMA+ myofibroblasts (**Fig. 22D,E**). Calcium imaging in ET1-treated epicardioid slices additionally revealed cardiomyocyte dysfunction across the myocardial layers, including frequent arrhythmic events and decreased calcium transient amplitudes, two well-established features of failing hearts (**Fig. 22F-H**).

### MATERIAL AND METHODS

The below disclosed materials and methods are one way of performing the inventive methods an uses. It is clear to the skilled persons that the specific cell types, specific compounds, supplements etc. can be replaced by surrogates from other manufacturers.

### Culture of human pluripotent stem cells

Human iPSCs were generated using the CytoTune-iPS 2.9 Sendai Reprogramming Kit (Invitrogen; A16157) as previously described. The following hiPSC lines were used in differentiation experiments: hPSCreg MRI003-A (hiPSC1), MRI001-A (hiPSC2), and MRI003-A-6 (AAVS1- CAG-VSFP; hiPSC3). Alternatively, hESC line HES-3 (hPSCreg ESIBle003) generated by ES Cell International Pte Ltd in Singapore was used.

Human pluripotent stem cells were cultured on Geltrex-coated plates (Gibco; A14133-02) in Essential 8 medium (Gibco; A1517001) containing 0.5% Penicillin/Streptomycin (Gibco; 15140-122). Cells were passaged every 4 days with 0.5 mM EDTA (Invitrogen; AM92606) in PBS without Ca2+ or Mg2+ (PBS-/-; Gibco; 10010023).

### Immunofluorescence analysis

For the production of cryosections, spheroids were washed with DPBS and fixed with 4% PFA (Sigma-Aldrich; 158127) for 1 h at RT. After washing 3 times with DPBS, they were kept in 30% sucrose at 4°C overnight. They were then embedded in a solution of 10% sucrose and 7.5% gelatin in DPBS before freezing in a 2-methyl-butane bath (Sigma-Aldrich; M32631) cooled with liquid nitrogen and transferring to -80°C. Cryosections prepared with a Microm HM 560 cryostat (Thermo Fisher Scientific, Germany) were transferred onto poly-L-lysine slides (Thermo Fisher Scientific; J2800AMNT) and stored at -80°C before immunofluorescence analysis.

For immunostaining, live cells were washed with DPBS and fixed with 4% PFA for 15 min at RT, while cryosections were fixed with 4% PFA for 10 min at RT. After washing 3 times with DPBS, cells or cryosections were permeabilized with 0.25% Triton X-100 (Sigma-Aldrich; X100) in DPBS for 15 min at RT. After washing another 3 times with DPBS, samples were blocked with 3% BSA in PBST, consisting of 0.05% Tween 20 (Sigma-Aldrich; P2287) in DPBS, for 1 hour at RT. Primary antibodies were then added at the indicated dilutions in 0.5% BSA in PBST and incubated overnight at 4°C. After washing 3 times for 5 min (cells) or 5 times for 10 min (cryosections) with PBST, appropriate secondary antibodies diluted in 0.5% BSA (Sigma-Aldrich; A9647) in PBST were added for 1 hour (cells) or 2 hours (cryosections) at RT, protected from light. After repeating the previous washing steps, Hoechst 33258 (Sigma-Aldrich; 94403) was added at a final concentration of 5 µg/mL in DPBS for 15 min at RT, protected from light. Samples were mounted with fluorescence mounting medium (Dako; S3023) and stored at 4°C until imaging with an inverted or confocal laser scanning microscope (DMI6000B and TCS SP8; Leica Microsystems, Wetzlar, Germany).

### Single-cell genomics

Epicardioids were dissociated to single cells using papain, adapting the number of pooled epicardioids and dissociation time to the stage of development. Briefly, a 2x papain solution consisting of 40 U/mL papain (Worthington Biochemical Corporation; LS003124) and 2 mM L-cysteine (Sigma-Aldrich; C6852) in PBS-/- was incubated 10 min at 37°C to activate the papain before 1:2 dilution in PBS-/- to obtain the 1× solution. Spheroids were then carefully pushed out of the collagen gel using 10 µL pipette tips if necessary and washed twice with 2 mM EDTA in PBS-/-. They were then dissociated in 750 µL 1× papain solution at 37°C and 750 rpm on a Thermomixer (Eppendorf, Germany). The enzymatic reaction was stopped with 750 µL stop solution consisting of 1 mg/mL trypsin inhibitor (Sigma-Aldrich; T9253) in PBS-/-. After pipetting up and down approximately 30 times to obtain a single cell suspension, cells were passed through a 40 µm strainer washed with 5 mL 1% BSA (Gibco;15260037) in PBS-/-. After centrifugation for 3 min at 200 g, cells were resuspended in 500 µL 0.5% BSA in PBS-/- for counting with Trypan blue. For samples exceeding 15% cell death, dead cells were immediately depleted using the Dead Cell Removal Kit (Miltenyi Biotec; 130-090-101) according to the manufacturer's instructions before further processing.

### Endothelin-1 treatment

To induce hypertrophy, epicardioids were treated with 25 nM or 50 nM endothelin-1 (Sigma-Aldrich; E7764) in maintenance medium for 6 days, replacing the medium every day. At the end of the treatment, they were dissociated with TrypLE Express (Gibco; 12605010) for 15 min at 37°C for RNA extraction or fixed with 4% PFA for 1 hour for cryosectioning. For cell size measurements, ET1-treated epicardioids and untreated controls were dissociated to single cells with papain as described above and re-seeded at a density of 25,000 cells/cm² coated with 2 µg/cm² fibronectin (Sigma-Aldrich; F1141). After 4 days, cells were fixed for immunofluorescence staining for cTnT as well as the desmosomal marker plakophilin-2 (PKP2) to visualize cell membranes. The area of cardiomyocytes was quantified in ImageJ (National Institutes of Health).

### Statistics

Statistical analysis was performed with GraphPad Prism version 9.1.0. (La Jolla California, USA). Box-and-whiskers plots indicate the median, 25th and 75th percentile, with whiskers extending to the 5th and 95th percentiles; bar graphs indicate the mean ± SEM and all data points, unless otherwise indicated. Normally distributed data from two experimental groups were compared by Student's t-test, otherwise a Mann-Whitney-Wilcoxon test was applied. Normally distributed data from more than two experimental groups were compared using one- or two-way analysis of variance (ANOVA). In the case of multiple comparisons, an appropriate post hoc test was applied as indicated. A p-value < 0.05 was considered statistically significant.

## Claims

1. Epicardioid comprising an inner myocardial compartment and an outer epicardial compartment,
wherein the outer epicardial compartment comprises from the outside to the inside
- a layer of epicardium comprising KRT18+ mesothelial epicardial cells, and
- a layer of subepicardium comprising Vim+ epicardial-derived cells (EPDCs); and wherein the inner myocardial compartment comprises from the outside to the inside
- a layer of compact-like myocardium comprising densely packed cTNT+ myocardial cells and
- a layer of trabecular-like myocardium comprising looser packed cTnT+ myocardial cells compared to the compact-like myocardium,
wherein the epicardioid has a three-dimensional shape,
wherein the myocardial compartment forms the inner core of the epicardioid and
wherein the epicardial compartment forms the envelope of the epicardioid,
wherein the epicardioid has been developed from one initial *in vitro* cell population.

2. Epicardioid according claim 1, wherein the initial *in vitro* cell population is an *in vitro* pluripotent stem cell population comprised in cell culture medium, wherein the pluripotent stem cell population is preferably an induced pluripotent stem cell population, more preferably a human induced pluripotent stem cell population, or wherein the pluripotent stem cell population is preferably an embryonic stem cell population, more preferably a human embryonic stem cell population.

3. Epicardioid according to claim 1 or 2, wherein the cells of the outer epicardial compartment further express epicardial transcription factor TBX18 and/or wherein the layer of compacted myocardium comprises a significantly higher proportion of Ki67+ proliferating cardiomyocytes than the inner trabecular-like layer of myocardium.

4. Epicardioid according to any one of the preceding claims, wherein the inner myocardial compartment further comprises in both layers non-cardiomyocyte cells comprising cardiac fibroblasts, vascular smooth muscle cells and endothelial cells.

5. Epicardioid according to any one of the preceding claims, wherein the two compartments of the epicardioid match the multilayered structure of the ventricular epicardium and myocardium of human embryos.

6. Use of an epicardioid according to any one of claims 1-5 as a heart tissue model.

7. Use of an epicardioid according to any one of claims 1-5 for studying heart diseases, preferably, left ventricular heart diseases, or for preclinical screening and testing of compounds or drugs for the treatment of heart diseases, wherein the heart diseases are preferably hypertrophic and/or fibrotic heart diseases, or for preclinical testing of drug-induced arrhythmias.

8. Method of producing a three-dimensional heart tissue model comprising
an inner myocardial compartment and an outer epicardial compartment,
wherein the outer epicardial compartment comprises from the outside to the inside
- a layer of epicardium comprising KRT18+ mesothelial epicardial cells, and
- a layer of subepicardium comprising Vim+ epicardial-derived cells (EPDCs); and wherein the inner myocardial compartment comprises from the outside to the inside
- a layer of compact-like myocardium comprising densely packed cTNT+ myocardial cells, and
- a layer of trabecular-like myocardium comprising looser packed cTnT+ myocardial cells compared to the compact-like myocardium,
wherein the epicardioid has a three-dimensional shape,
wherein the myocardial compartment forms the inner core of the epicardioid and
wherein the epicardial compartment forms the envelope of the epicardioid,
wherein the method comprises the steps of:
- providing pluripotent stem cells (PCSs) and seeding the PSCs in an initial culture medium on day -1 of the culture,
- replacing the culture medium with a first basal differentiation medium on day 0 of the culture,
- replacing the culture medium with a second basal differentiation medium comprising retinoic acid on day 2 of the culture,
- refreshing the culture medium with the second basal differentiation medium each 24 hours until day 6 of the culture,
- replacing the culture medium with a third basal differentiation medium on day 6 of the culture,
- refreshing the culture medium with the third basal differentiation medium on day 7 of the culture,
- embedding spheroids into a gel comprising collagen type I, and transferring the gel sheets to maintenance medium on day 8 of the culture,
- optionally placing the gel sheets on a rocking shaker on day 10 of the culture,
- replacing maintenance medium every 2-3 days after day 8,
- isolating epicardioids for further use between days 13 and 100 of the culture, preferably between days 15 and 40.

9. Method of claim 8, wherein the heart tissue model produced by the method is an epicardioid according to claims 1-5.

10. Method of claim 8 or 9, wherein the PSCs are human pluripotent stem cells,
preferably human induced pluripotent stem cells (hiPSCs), and/or wherein the PSCs are embryonic stem cells, preferably human embryonic stem cells,
and optionally,
wherein the initial number of PSCs on day -1 of the culture is 30,000-40,000 per well of a 96-well plate,
and optionally,
wherein the 96-well plate comprises U-shaped wells which are optionally coated with a low cell adherence agent, preferably with poly-HEMA.

11. Method of any one of claims 8-10, wherein the retinoic acid in the second basal differentiation medium is present in an amount of 0.3-0.75 µM, preferably 0,5 µM; and/or
wherein the initial culture medium comprises ROCK inhibitor thiazovivin, preferably
wherein the initial culture medium is Essential 8 medium comprising 1-5 µM, preferably 2 µM thiazovivin;
and/or
wherein the first basal medium comprises BMP4, Activin A, bFGF, a PI3 kinase inhibitor, preferably LY-29004, and a Wnt activator, preferably CHIR-99021, and/or
wherein the second basal medium further comprises insulin, BMP4, bFGF, and a Wntantagonist, preferably IWP2;
and/or
wherein the third basal medium comprises insulin, BMP4, bFGF;
and/or
wherein the maintenance medium comprises VEGF.

12. Method for screening or testing a candidate compound for its effects on heart development and/or functionality comprising producing a heart tissue model according to the method of any one of claims 8-11 while treating the cells with the candidate compound and comparing development of the heart tissue model with development and/or functionality of a heart tissue model that was not treated with the candidate compound.

13. Method of claim 12, wherein the compound to be tested is added to the culture either during culture days 1 to 13, or after day 13.

14. A heart tissue model prepared according to the method of any one of claims 8-11.

15. The heart tissue model according to claim 14, wherein the heart tissue model comprises
an inner myocardial compartment and an outer epicardial compartment,
wherein the outer epicardial compartment comprises from the outside to the inside
- a layer of epicardium comprising KRT18+ mesothelial epicardial cells, and
- a layer of subepicardium comprising Vim+ epcardial-derived cells (EPDCs); and wherein the inner myocardial compartment comprises from the outside to the inside
- a layer of compact-like myocardium comprising densely packed cTNT+ myocardial cells, and
- a layer of trabecular-like myocardium comprising looser packed cTnT+ myocardial cells compared to the compact-like myocardium,
wherein the epicardioid has a three-dimensional shape,
wherein the myocardial compartment forms the inner core of the epicardioid and wherein the epicardial compartment forms the envelope of the epicardioid;
or
wherein the heart tissue model has been isolated from culture before day 13 of the culture.
